# EUROPEAN PATENT APPLICATION

(11) **EP 4 650 346 A1**
(43) Date of publication of application: **19.11.2025**
(21) Application number: 23922538.6
(22) Date of filing: 27.12.2023
(51) Int. Cl.: C07D 209/88, C07D 209/86, C07C 211/54

(54) **ORGANIC COMPOUND, AND PREPARATION METHOD THEREFOR AND USE THEREOF**

(30) Priority: 16.02.2023 CN 202310128732
(71) Applicant: Contemporary Amperex Future Energy Research Institute (Shanghai) Limited, Shanghai 200241 (CN); Contemporary Amperex Technology Co., Limited, Ningde, Fujian 352100 (CN)
(72) Inventor: JIA, Boyu, Shanghai 200241 (CN); LIANG, Weifeng, Shanghai 200241 (CN); CHEN, Guodong, Shanghai 200241 (CN); GUO, Yongsheng, Shanghai 200241 (CN)
(74) Representative: Kraus & Lederer PartGmbB
(86) International application number: PCT/CN2023/142513
(87) International publication number: WO 2024/169423

(57) **Abstract**

An organic compound and a preparation method thereof and use thereof are provided. The organic compound includes head groups, a tail group, and a carbon chain, where the carbon chain connects the head groups to the tail group, and there are at least two head groups. The organic compound has high bonding strength with a substrate surface and a high content, thereby effectively improving completeness of a monomolecular film layer formed by the tail group contained in the organic compound on the substrate surface, enhancing bonding strength and stability of the film layer formed by the organic compound and the substrate, effectively improving matching between energy levels of an electrode and a perovskite layer, and enhancing photoelectric performance such as an open-circuit voltage and photoelectric conversion efficiency of a perovskite solar cell.

## Description

This application claims priority to Chinese Patent Application No. 202310128732.8, filed with the China National Intellectual Property Administration on February 16, 2023, entitled "ORGANIC COMPOUND AND PREPARATION METHOD THEREOF AND USE THEREOF", which is incorporated herein by reference in its entirety.

### TECHNICAL FIELD

This application relates to the technical field of solar cells, and specifically, to an organic compound and a preparation method thereof, a hole transport material, a perovskite solar cell, and an electric apparatus.

### BACKGROUND

A perovskite solar cell contains a hole transport layer, and this hole transport layer can cause a decrease in a work function of a positive electrode, to match an energy level of a perovskite layer, thereby increasing an open-circuit voltage of the perovskite solar cell. However, existing hole transport materials and an electrode have low bonding strength and form a poor film layer, which suppresses charge extraction from the perovskite solar cell, thereby significantly decreasing photoelectric conversion efficiency of the perovskite solar cell.

### TECHNICAL PROBLEM

In view of the foregoing problems, embodiments of this application provide an organic compound and a preparation method thereof and use thereof, to resolve a technical problem that a monomolecular film layer is incomplete and easy to fall off because self-assembled molecules used as a hole transport material has low bonding strength for a positive electrode in the prior art.

### TECHNICAL SOLUTION

According to a first aspect, an embodiment of this application provides an organic compound. The organic compound in this embodiment of this application includes head groups, a tail group, and a carbon chain, where the carbon chain connects the head groups to the tail group, and there are at least two head groups.

The number of head groups of the organic compound in this embodiment of this application is increased to enhance bonding strength and a bonding volume of the organic compound for a substrate surface and improve distribution uniformity of the organic compound on the substrate, thereby effectively improving completeness of a monomolecular film layer formed by the tail group contained in the organic compound in this embodiment of this application on the substrate surface, enhancing bonding strength of the film layer formed by the organic compound for the substrate, increasing stability of the monomolecular film layer, and improving uniformity of the monomolecular film layer. In addition, the head groups can further exert a polarity adjustment effect on the organic compound together with the tail group and the carbon chain, thereby fully exerting a function of forming a hole transport layer by the organic compound in this embodiment of this application.

In some embodiments, the at least two head groups are identical or different and include at least two of -SO₃H, -PO(OH)₂, -COOH, and -Si(ORₐ)₃, or at least two salts of -SO₃H, -PO(OH)₂, and -COOH, where Rₐ represents a first alkyl group.

In an embodiment, the first alkyl group is a C1-C5 alkyl group.

Selection of these specific head groups can further enhance the bonding strength and the bonding volume of the organic compound for the substrate surface as well as distribution uniformity, thereby further improving completeness and stability of the monomolecular film layer formed by the tail group contained in the organic compound in this embodiment of this application on the substrate surface. In addition, the head groups can further adjust polarity of the organic compound in this embodiment of this application, thereby further exerting a function of forming the hole transport layer by the organic compound in this embodiment of this application.

In some embodiments, the tail group includes at least one group selected from substituted or unsubstituted carbazole, substituted or unsubstituted cyclopentadithiophene, substituted or unsubstituted benzodithiophene, substituted or unsubstituted pyrrolodithiophene, substituted or unsubstituted diphenylamine, and substituted or unsubstituted triphenylamine.

These tail groups represent aromatic groups or aromatic heterocyclic groups, have relatively high hydrophobicity, form a hydrophobic end, and have a π-π interaction effect, which enhances self-assembly of the organic compound in this embodiment of this application into a monomolecular layer through the foregoing types of tail groups. In addition, these groups can adjust polarity of the organic compound in this embodiment of this application together with the head groups as well as the carbon chain, thereby further increasing a bandwidth of the organic compound in this embodiment of this application and enhancing extraction of carriers.

In an embodiment, the carbazole group is 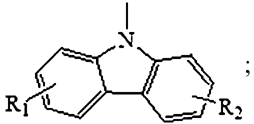 the cyclopentadithiophene group is 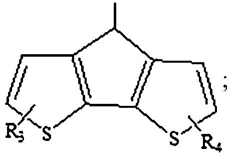 the benzodithiophene group is 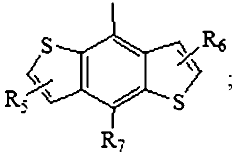 the pyrrolodithiophene group is 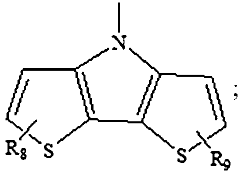 the diphenylamine group is 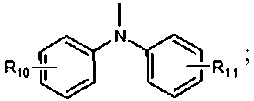 and the triphenylamine group is 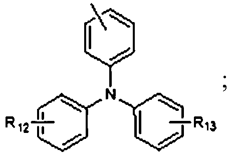 where R₁, R₂, R₃, R₄, R₅, R₆, R₇, R₈, R₉, R₁₀, R₁₁, R₁₂, and R₁₃ independently include any one of hydrogen, halogen, -O-R_{b}, -OH, -NHCOR_{c}, -OCOR_{d}, -CH₂COOH, -Rₑ, a phenyl group, a halogen-substituted phenyl group, halogen-substituted R_{f}, a nitrogen-containing group, nitrogen-containing group-substituted R_{g}, and a nitrogen-containing group-substituted phenyl group, where R_{b}, R_{c}, R_{d}, Rₑ, R_{f}, and R_{g} independently represent a second alkyl group.

In an embodiment, the second alkyl group is a C1-C5 alkyl group.

In an embodiment, the nitrogen-containing group includes any one of-N(Rₕ)₂, -NHRᵢ, -NH₂, a trimethylamino group, a triethylamino group, and a tripropylamino group, where Rₕ and Rᵢ independently represent a third alkyl group.

The substituted or unsubstituted tail groups in the foregoing embodiments all have relatively high hydrophobicity and π-π interaction, which can further enhance self-assembly of the organic compound in the foregoing embodiments into a monomolecular layer via these tail groups, adjust the polarity of the organic compound in this embodiment of this application, widen a bandgap of the organic compound in the foregoing embodiments, and improve extraction of carriers.

In some embodiments, the carbon chain includes at least one of an alkyl chain and a heteroatom-containing alkyl chain.

In an embodiment, the number of carbon atoms in the alkyl chain ranges from C1 to C10.

In an embodiment, the heteroatom includes at least one of O, S, N, B, and Si.

These carbon chains can adjust polarity of the organic compound in this embodiment of this application together with the head groups and the foregoing tail groups, which can further improve match between a work function of an electrode, particularly a conductive oxide electrode, and an energy level of a perovskite layer interface, reduce energy loss, and increase an open-circuit voltage of a perovskite cell, thereby significantly increasing photoelectric conversion efficiency of the perovskite solar cell.

In some embodiments, one end of the carbon chain is connected to the head group, and another end of the carbon chain is connected to the tail group.

Connecting the head group and the tail group to two ends of the carbon chain can effectively reduce steric hindrance of the organic compound in this embodiment of this application, improve self-assembly of the tail group into a monomolecular layer, adjust and improve stacking orientation of the tail group and the head group, and increase stability of molecules of the organic compound in this embodiment of this application.

In some embodiments, the organic compound includes at least one of the following compounds or salts of the compounds: 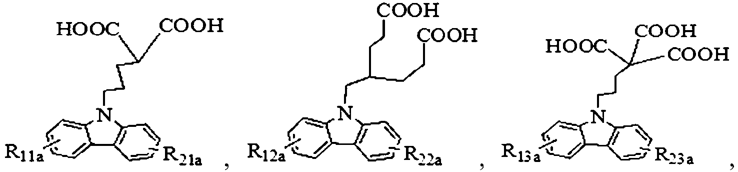 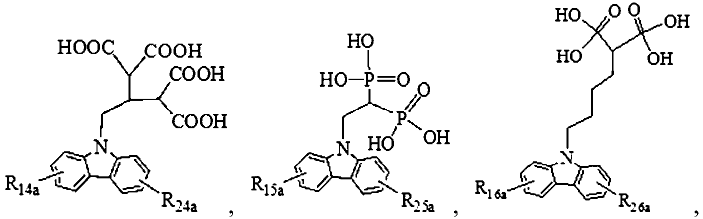 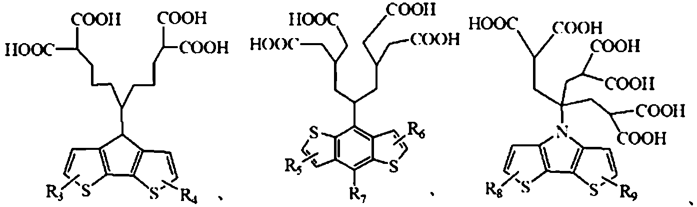 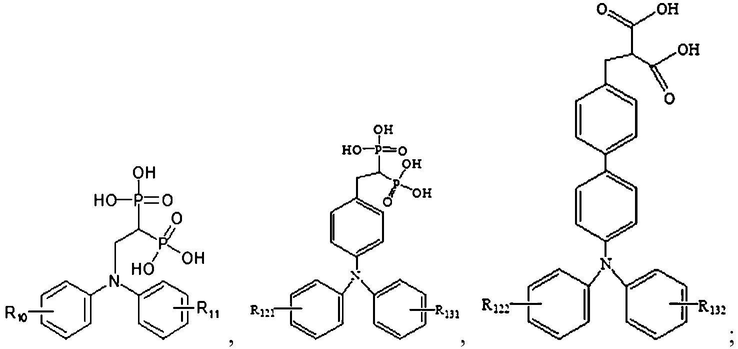 where R₁₁ₐ, R₁₂ₐ, R₁₃ₐ, R₁₄ₐ, R₁₅ₐ, and R₁₆ₐ independently represent R₁, R₂₁ₐ, R₂₂ₐ, R₂₃ₐ, R₂₄ₐ, R₂₅ₐ, and R₂₆ₐ independently represent R₂, R₁₂₁ and R₁₂₂ independently represent R₁₂, and R₁₃₁ and R₁₃₂ independently represent R₁₃.

The organic compound denoted as the foregoing specific chemical formula contains a plurality of head groups, which can enhance bonding strength and bonding volume of the organic compound for the substrate and improve completeness of the formed monomolecular film layer. In addition, selection and assembly design of the head groups, carbon chain, and tail group for the organic compound denoted as the foregoing specific chemical formula can further improve match between a work function of an electrode, particularly a conductive oxide electrode, and an energy level of a perovskite layer interface, and reduce energy loss and an open-circuit voltage of a perovskite cell, thereby significantly enhancing photoelectric conversion efficiency of the perovskite solar cell.

According to a second aspect, an embodiment of this application provides a preparation method of an organic compound. The preparation method of the organic compound in this embodiment of this application includes the following steps:
subjecting a reactant Fₐ containing a tail group and a reactant F_{b} containing a first carbon chain to a coupling reaction, to connect the tail group to the first carbon chain to generate an intermediate product Cₐ; and
subjecting the intermediate product Cₐ and a reactant F_{c} containing a first ester group to a substitution reaction, where the first ester group is an ester group containing a first head group, so that the first head group is connected to the first carbon chain to generate an intermediate product C_{b};
   or
subjecting a reactant Fₐ containing a tail group and a reactant Fₑ containing a second carbon chain and a second ester group to an addition reaction, to connect the tail group to the second carbon chain to generate an intermediate product C_{c}, where the second ester group is an ester group containing a second head group, and the second head group is connected to the second carbon chain; and
subjecting the first ester group contained in the intermediate product C_{b} and/or the second ester group contained in the intermediate product C_{c} to a hydrolysis reaction to generate a final product of the organic compound;
where the final product of the organic compound contains at least two head groups.

In the preparation method of the organic compound in this embodiment of this application, the tail group and at least two head groups can be effectively connected to the carbon chain, to form the organic compound containing a plurality of head groups in the foregoing embodiment of this application, so that the prepared organic compound has high bonding strength and a great bonding volume for a substrate surface, which improves completeness, stability, and uniformity of a monomolecular film layer formed by the tail group contained in the organic compound on the substrate surface, and can also adjust the head groups, the carbon chain, and the tail group to adjust polarity of the organic compound, thereby fully exerting an effect of forming a hole transport layer by the organic compound. In addition, the preparation method of the organic compound in this embodiment of this application features easily controllable reaction conditions, few byproducts, and a high yield of the final product.

In some embodiments, the reactant Fₐ includes at least one of the following compounds: 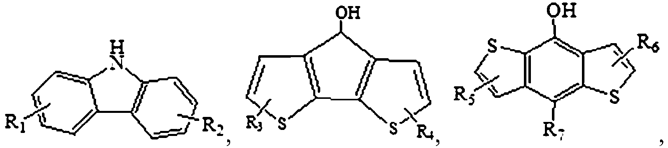 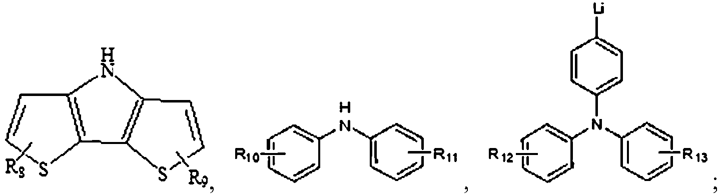 where R₁, R₂, R₃, R₄, R₅, R₆, R₇, R₈, R₉, R₁₀, R₁₁, R₁₂, and R₁₃ independently include any one of hydrogen, halogen, -O-R_{b}, -OH, -NHCOR_{c}, -OCOR_{d}, -CH₂COOH, -Rₑ, a phenyl group, a halogen-substituted phenyl group, halogen-substituted R_{f}, a nitrogen-containing group, nitrogen-containing group-substituted R_{g}, and a nitrogen-containing group-substituted phenyl group, where R_{b}, R_{c}, R_{d}, Rₑ, R_{f}, and R_{g} independently represent a fourth alkyl group.

In some embodiments, the reactant F_{b} includes any one of a haloalkane compound, a heteroatom-containing haloalkane compound, and a halogenated alkyl acyl halide compound.

In some embodiments, the reactant F_{c} includes a compound containing at least two of an ester group of -SO₃H, an ester group of -PO(OH)₂, an ester group of-COOH, and an ester group of -Si(ORₐ)₃; where Rₐ represents a fifth alkyl group.

In some embodiments, the reactant Fₑ includes any one of a heteroatom-containing haloalkane compound and a halogenated compound containing at least one of an ester group of -SO₃H, an ester group of -PO(OH)₂, an ester group of -COOH, and -Si(ORₐ)₃ and any one of an alkyl chain and a heteroatom-containing alkyl chain, where Rₐ represents a sixth alkyl group.

In an exemplary embodiment, the reactant Fₑ includes at least one of the following compounds: 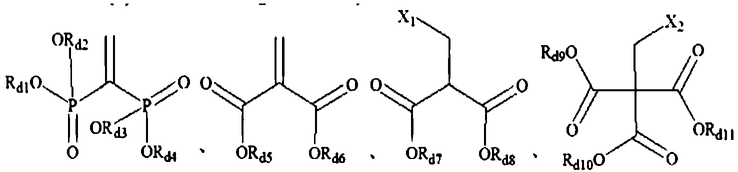 where R_{d1}, R_{d2}, R_{d3}, R_{d4}, R_{d5}, R_{d6}, R_{d7}, R_{d8}, R_{d9}, R_{d10}, and R_{d11} independently represent a C1-C5 alkyl chain, and X₁ and X₂ independently represent a halogen atom.

Through selection of each reactant, the number of head groups contained in the final product of the organic compound can be further adjusted, and when the organic compound is used as the hole transport material, action forces of the hole transport layer and the substrate can be further enhanced, thereby improving uniformity of self-assembled molecules on the substrate, improving stability of a thin film formed by the final product of the organic compound used as the hole transport material, and enhancing stability of a perovskite solar cell. In addition, performance such as polarity of the final product of the organic compound can be adjusted, to effectively improve match between a work function of an electrode, particularly a conductive oxide electrode, and an energy level of a perovskite layer interface, and an open-circuit voltage of a perovskite cell, and reduce energy loss, thereby significantly improving photoelectric conversion efficiency of the perovskite solar cell.

In some embodiments, solvents for the coupling reaction and the substitution reaction independently include at least one of N,N-dimethylformamide, toluene, water, 1,2-xylene, chlorobenzene, 1,2-dichlorobenzene, tetrahydrofuran, and ethanol.

In some embodiments, a solvent for the hydrolysis reaction independently includes at least one of 1,4-dioxane, water, anhydrous ethanol, tetrahydrofuran, toluene, 1,2-xylene, chlorobenzene, and 1,2-dichlorobenzene.

In some embodiments, a solvent for the addition reaction includes at least one of tetrahydrofuran, anhydrous ethanol, toluene, 1,2-xylene, chlorobenzene, and 1,2-dichlorobenzene.

Through selection of solvents for the coupling reaction, substitution reaction, addition reaction, and hydrolysis reaction systems, reaction efficiency of the coupling reaction, substitution reaction, addition reaction, and hydrolysis reaction can be improved, thereby increasing a yield of a target product.

According to a third aspect, an embodiment of this application provides a hole transport material. The hole transport material in this embodiment of this application contains the organic compound in the foregoing embodiment of this application or the organic compound prepared in the preparation method in the foregoing embodiment of this application.

The hole transport material in this embodiment of this application has high bonding strength for an electrode, and can form a monomolecular layer on a surface of the electrode and also have a wide bandgap, which can effectively improve match between a work function of an electrode, particularly a conductive oxide electrode, and an energy level of a perovskite layer interface, improve extraction of carriers, reduce energy loss, and increase an open-circuit voltage of a perovskite cell, thereby significantly enhancing photoelectric conversion efficiency of a perovskite solar cell.

According to a fourth aspect, an embodiment of this application provides a perovskite solar cell. The perovskite solar cell in this embodiment of this application includes a hole transport layer, and the hole transport layer contains the organic compound in the foregoing embodiment of this application, the organic compound prepared in the preparation method in the foregoing embodiment of this application, or the hole transport material in the foregoing embodiment of this application.

A material contained in the hole transport layer of the perovskite solar cell in this embodiment of this application has a wide bandgap, which can effectively improve match between a work function of an electrode, particularly a conductive oxide electrode, and an energy level of a perovskite layer interface, significantly improve extraction of carriers, reduce energy loss, and increase an open-circuit voltage of a perovskite cell, thereby significantly enhancing photoelectric conversion efficiency of a perovskite solar cell.

In some embodiments, a thickness of the hole transport layer ranges from 0.1 nm to 10 nm.

In some embodiments, the hole transport layer is stacked between a conductive oxide electrode and a perovskite layer contained in the perovskite solar cell.

In some embodiments, perovskite contained in the perovskite layer of the perovskite solar cell includes at least one of ABX₃ and A₂CDX₆, where A, B, C, and D independently and differently represent an inorganic or organic cation or a mixture of organic and inorganic cations, and X represents an inorganic or organic anion or a mixture of organic and inorganic anions.

In some embodiments, a material of a conductive oxide electrode contained in the perovskite solar cell includes at least one of a transparent material of a fluorine-doped tin oxide, indium tin oxide, a transparent conductive material of an aluminum-doped zinc oxide, a transparent conductive material of a boron-doped zinc oxide, and a transparent conductive material of an indium-doped zinc oxide.

Through further selection and control of a thickness of the hole transport layer, a material of the perovskite layer, a stacking position of the perovskite layer in the perovskite solar cell, and a material of the conductive oxide electrode, match between a work function of an electrode, particularly a conductive oxide electrode, and an energy level of a perovskite layer interface can be further improved, extraction of carriers is improved and energy loss is reduced, thereby significantly enhancing photoelectric conversion efficiency of a perovskite solar cell.

According to a fifth aspect, an embodiment of this application provides a preparation method of a perovskite solar cell. The preparation method of the perovskite solar cell in this embodiment of this application includes the following steps:
providing a first electrode;
preparing a slurry including an organic compound or a hole transport material, and subjecting the slurry to film-forming processing on a surface of the first electrode to form a hole transport layer;
forming a perovskite layer on a surface, facing away from the first electrode, of the hole transport layer;
forming an electron transport layer on a surface, facing away from the hole transport layer, of the perovskite layer; and
forming a second electrode on a surface, facing away from the perovskite layer, of the electron transport layer;
where the organic compound includes the organic compound in the embodiments of this application or the organic compound prepared in the preparation method of the organic compound in the embodiments of this application, and the hole transport material includes the hole transport material in the embodiments of this application.

In the preparation method of the perovskite solar cell in this embodiment of this application, the slurry including the organic compound in the foregoing embodiment of this application is used to form the hole transport layer on the surface of the first electrode, and in this way, a head group of the organic compound included in the hole transport layer can be effectively bonded to the surface of the first electrode, and the tail group included in the organic compound can be self-assembled into a monomolecular film layer, thereby fully exerting the foregoing effects of the organic compound in the foregoing embodiment of this application, and enhancing photoelectric conversion efficiency of the perovskite solar cell in this embodiment of this application. In addition, because the formed hole transport layer has large bonding strength for the surface of the first electrode, when the perovskite layer is formed on the surface of the hole transport layer, the hole transport layer can resist erosion of a perovskite precursor solution, thereby ensuring stability of the hole transport layer, that is, completeness and stability of the monomolecular layer formed on the surface of the first electrode.

In some embodiments, the first electrode is a conductive oxide electrode. In this way, the formed hole transport layer can be directly stacked onto and bonded with the conductive oxide electrode layer, to enhance chemical adsorption of the organic compound in the foregoing embodiment of this application in the slurry to the conductive oxide electrode layer, thereby further improving bonding strength of the hole transport layer for the conductive oxide electrode and enhancing energy level match between the conductive oxide electrode and the perovskite layer.

In some embodiments, a concentration of the organic compound or the hole transport material in the slurry ranges from 0.1 mg/mL to 10 mg/mL. A slurry in this concentration range can ensure that a head group of the organic compound in this embodiment of this application is sufficiently bonded to a surface of the first electrode such as a conductive oxide, and a tail group is sufficiently self-assembled into an integral monomolecular film layer.

According to a sixth aspect, an embodiment of this application provides an electric apparatus. The electric apparatus provided in this embodiment of this application includes the foregoing perovskite solar cell in this application or the foregoing perovskite solar cell prepared in the preparation method of the perovskite solar cell in this application, where the perovskite solar cell serves as a power supply or an energy storage unit of the electric apparatus.

Because the electric apparatus includes the perovskite solar cell in this embodiment of this application, the power supply provides more stable electric energy, operating time is prolonged, or energy storage efficiency is increased.

The foregoing description is merely an overview of the technical solutions in this application. To more clearly understand the technical means in this application, achieve implementation according to the content of this specification, and make the foregoing and other objectives, features, and advantages of this application more obvious and comprehensible; specific embodiments of this application are described below.

### BRIEF DESCRIPTION OF DRAWINGS

After reading detailed descriptions of the following preferred embodiments, a person of ordinary skill in the art can clearly understand other advantages and benefits. The accompanying drawings are only used to illustrate the preferred embodiments rather than be considered as a limitation on this application. In addition, in all drawings, identical components are denoted with identical reference numerals. The accompanying drawings are as follows:
FIG. 1 is a schematic structural diagram of an existing type-p-i-n perovskite solar cell;
FIG. 2 is a schematic diagram of energy levels of a perovskite (PVSK) layer and a transparent conductive oxide (TCO) electrode in existing perovskite solar cells, where FIG. a is a schematic diagram of energy levels of a perovskite (PVSK) layer and a transparent conductive oxide (TCO) electrode contained in a perovskite solar cell without a hole transport layer; and FIG. b is a schematic diagram of energy levels of a PVSK and a TCO after addition of a self-assembled monomolecular layer; and
FIG. 3 is a schematic flowchart of a preparation method of an organic compound according to an embodiment of this application.

### DESCRIPTION OF EMBODIMENTS

Embodiments of the technical solutions in this application are described in detail below with reference to the accompanying drawings. The following embodiments are only used to illustrate the technical solutions in this application more clearly, and therefore, are used only as examples, and cannot be used to limit the protection scope of this application.

Unless otherwise defined, all technical and scientific terms used herein have the same meanings as commonly understood by a person skilled in the technical field of this application; terms used herein are only used to describe specific embodiments rather than limit this application; and the terms "include", "have" and any variations thereof in the specification, claims, and descriptions of the foregoing accompanying drawings in this application are intended for non-exclusive inclusion.

In the descriptions of the embodiments of this application, technical terms such as "first" and "second" are only used to differentiate different objects and shall not be understood as indication or implication of relative importance or implicit indication of the number, a specific order, or a subordination relationship of indicated technical features. In the descriptions of the embodiments of this application, unless otherwise explicitly and specifically limited, "a plurality of" means "more than two".

Reference to "embodiment" herein means that specific features, structures, or characteristics described with reference to the embodiment may be included in at least one embodiment of this application. Such phrase appearing in various places in this specification does not necessarily refer to the same embodiment or an independent or alternative embodiment that is exclusive of other embodiments. A person skilled in the art can explicitly and implicitly understand that the embodiments described herein can be combined with other embodiments.

In the descriptions of the embodiments of this application, the term "and/or" describes only an association relationship for describing associated objects and indicates that three relationships may exist. For example, A and/or B may represent the following three cases: Only A exists, both A and B exist, and only B exists. In addition, a character "/" in this specification generally indicates an "or" relationship between contextually associated objects.

In the descriptions of the embodiments of this application, the term "a plurality of' means "two (inclusive) or more than two". Similarly, "a plurality of groups" means two (inclusive) or more than two groups, and "a plurality of pieces" means two (inclusive) or more than two pieces.

In the descriptions of the embodiments of this application, the orientations or positional relationships indicated by the technical terms "center", "longitudinal", "transverse", "length", "width", "thickness", "upper", "lower", "front", "rear", "left", "right", "perpendicular", "horizontal", "top", "bottom", "inner", "outer", "clockwise",, "anti-clockwise" "axial" "radial", "circumferential", and the like are based on the orientations or positional relationships shown in the accompanying drawings, are merely intended to facilitate the descriptions of this application and simplify the descriptions, are not intended to indicate or imply that the apparatuses or components mentioned in this application must have specific orientations, or be constructed and operated for a specific orientation, and therefore shall not be construed as a limitation on the descriptions of this application.

In the descriptions of the embodiments of this application, unless otherwise specified and defined explicitly, the technical terms "mount", "connect", "join", and "fix" should be understood in general senses. For example, the technical terms may refer to a fixed connection, a detachable connection, an integral connection, a mechanical connection, an electric connection, a direct connection, an indirect connection via an intermediate medium, an internal connection between two elements, or an interaction relationship between two elements. A person of ordinary skill in the art can understand specific meanings of the foregoing terms in the embodiments of this application based on specific situations.

Because a perovskite material has much greater light absorption performance than crystalline silica and undergoes low energy loss in an energy conversion process, and can still implement high-energy conversion under indoor or feeble illumination conditions, conversion efficiency of perovskite solar cells has increased rapidly over the past decade. In particular, type-p-i-n perovskite cells have advantages such as a simple structure, a small hysteresis effect, and good long-term stability without doping, and therefore, are very advantageous in future actual application. Therefore, application of the perovskite solar cells have gradually expanded. In addition, some companies have achieved mass production of the perovskite solar cells by now.

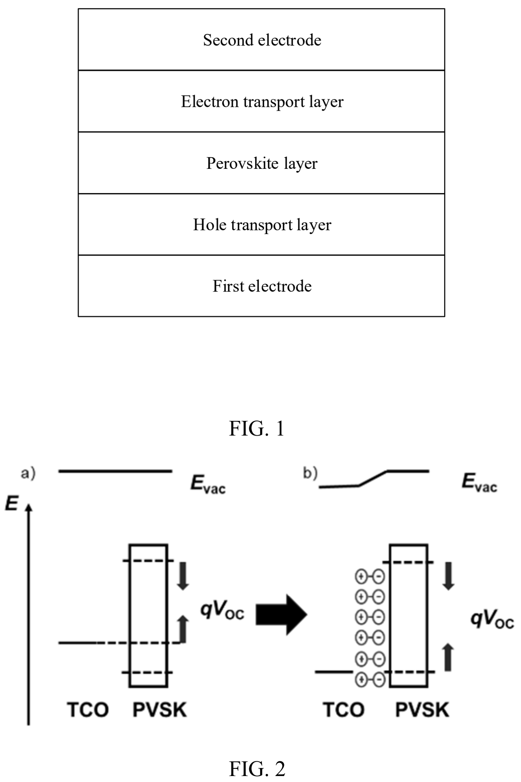

A structure of the perovskite solar cell generally includes a first electrode, a hole transport layer, a perovskite light absorption layer, an electron transport layer, and a second electrode sequentially stacked, and may specifically be a structure shown in FIG. 1. The hole transport layer significantly affects morphology of a perovskite active layer in a type-p-i-n cell and cell performance.

Self-assembled molecules have low preparation costs, can be prepared in solutions, and have good stability and reusability when used as a material for the hole transport layer in the type-p-i-n perovskite solar cells. In addition, the self-assembled molecules can improve energy level match between a positive electrode and the perovskite layer, and therefore, have significant commercial potential in the field of perovskite solar cells. In addition, the self-assembled molecules can form a uniform monomolecular self-assembled thin film on a substrate surface with the same molecular orientation and the same dipole vector direction, and generate a weak electric field that affects energy level structures of upper and lower interfaces. Therefore, a dipole of the self-assembled molecules can regulate a surface work function of an electrode such as a conductive metal oxide electrode, and is a key factor affecting energy loss of the perovskite solar cell. As shown in FIG. 2, FIG. a in FIG. 2 shows energy level structures of perovskite (PVSK) and a transparent conductive oxide (TCO) electrode without a hole transport layer, where an open-circuit voltage (V_{OC}) is a difference between the work function of the TCO and a quasi-Fermi level of the perovskite; and as shown in FIG. b in FIG. 2, after a self-assembled monomolecular layer is added, the work function of the TCO is decreased to become closer to the quasi-Fermi level of the PVSK, thereby increasing V_{OC} of the device.

The self-assembled molecules refer to an organic compound containing a head group, a tail group, and a carbon chain connecting the head group to the tail group. Although the self-assembled molecules have been reported by now and also include head groups at a hydrophilic end, after study, the inventor has found that existing disclosed self-assembled molecules have low bonding strength for the surface of the electrode such as the conductive oxide electrode, and are susceptible to subsequent processes. For example, the existing disclosed self-assembled molecules are easy to wash off with a perovskite precursor solution, and as a result, content of the self-assembled molecules on the surface of the electrode is low and it is difficult to form an integral monomolecular film layer. That is, the monomolecular film layer formed on the surface of the electrode is incomplete. For example, the monomolecular film layer with discontinuous or island-like morphology is formed. In addition, the formed incomplete monomolecular film layer has low bonding strength with the electrode and is likely to fall off, and as a result, the perovskite layer directly comes into contact with the electrode, which makes it difficult for the hole transport layer to exert its function, thereby suppressing extraction of charge from the perovskite cell and causing an insignificant increase in the open-circuit voltage of the perovskite solar cell, great energy loss, and a significant decrease in the photoelectric conversion efficiency.

To overcome the problem of low bonding strength of the existing self-assembled molecules for the surface of the electrode such as the conductive oxide electrode, the inventor conducted study and has found that designing a head group of the self-assembled molecule, for example increasing the number of head groups or further designing types of the head groups, can effectively enhance hydrophilicity of the self-assembled molecules and their bonding strength for the surface of the electrode, which improves bonding stability and content of the self-assembled molecules on the surface of the electrode, enhances completeness of a monomolecular film layer formed on the surface of the electrode, and increases stability of the monomolecular film layer, thereby fully exerting effects of forming the hole transport layer by the self-assembled molecules, for example adjustment of energy level match between the electrode and the perovskite layer and increase in the open-circuit voltage of the perovskite solar cell.

Further, after the study, the inventor has found that, on the basis of the addition of the head groups to enhance bonding strength of the self-assembled molecules for the electrode, the type design of the head group, tail group, and carbon chain of the self-assembled molecules can be further adjusted, to further adjust polarity of the self-assembled molecules and improve energy level match between the electrode and the perovskite layer, thereby further enhancing the open-circuit voltage, reducing voltage loss, and improving the photoelectric conversion efficiency of the perovskite cell.

Based on the study of the inventor, the following solutions are proposed.

### Organic Compound

According to a first aspect, an embodiment of this application provides an organic compound. The organic compound in this embodiment of this application includes head groups, a tail group, and a carbon chain, where the carbon chain connects the head group to the tail group, and there are at least two head groups.

Because the organic compound in this embodiment of this application includes the head groups, the tail group, and the carbon chain connecting the head groups to the tail group, the organic compound in this embodiment of this application belongs to organic self-assembled molecules. The carbon chain connects the head groups to the tail group through chemical bonds.

Because the organic compound in this embodiment of this application include at least two head groups, the at least two head groups endow the organic compound in this embodiment of this application with good hydrophilicity. With a relatively large number of head groups, an amount of chemical bonds formed on a surface of the substrate, particularly a conductive oxide substrate, is increased, which enhances an adsorption effect of chemical bonds between the organic compound and the substrate, thereby increasing bonding strength of the organic compound for the surface of the substrate such as the conductive substrate, effectively increasing a bonding volume of the organic compound on the surface of the substrate, improving distribution uniformity of the organic compound on the substrate, and effectively improving completeness of a monomolecular film layer formed by the organic compound in this embodiment of this application on the surface of the substrate. In addition, the bonding strength of the film layer formed by the organic compound for the substrate is enhanced, stability of the monomolecular film layer is improved, and the uniformity of the monomolecular film layer is improved. In addition, the head groups can further adjust the polarity of the organic compound together with the tail group and the carbon chain, thereby fully exerting the effect of forming the hole transport layer by the organic compound in this embodiment of this application, for example, effective enhancement of energy level match between the electrode, particularly the conductive oxide electrode, and the perovskite layer, and enhancement of photoelectric performance such as the open-circuit voltage and the photoelectric conversion efficiency of the perovskite solar cell.

In an embodiment, the at least two head groups contained in the organic compound in this embodiment of this application are identical or different and include at least two of -SO₃H, -PO(OH)₂, -COOH, and -Si(ORₐ)₃, or at least two salts of -SO₃H, -PO(OH)₂, and -COOH, where Rₐ represents a first alkyl group. Rₐ represents an alkyl group and is defined herein as the first alkyl group.

These selected specific head groups have good hydrophilicity and the number of head groups in the organic compound in this embodiment of this application is controlled to be at least two, which can further enhance contact between the organic compound and the surface of the substrate and formation of chemical bonds. Specifically, when the head group contains -SO₃H, -SO₃H can form a chemical bond of -S-O-M with the substrate; when the head group contains -PO(OH)₂, -PO(OH)₂ can form a chemical bond of -P-O-M with the substrate; when the head group contains-COOH, -COOH can form a chemical bond of -C-O-M with the substrate; and when the head group contains -Si(ORₐ)₃, the -Si(ORₐ)₃ can form a chemical bond of -Si-O-M with the substrate, where M includes a metal element and O represents oxygen. Therefore, these types of at least two head groups effectively enhance bonding strength of the organic compound for the surface of the substrate, such as a conductive substrate, that is, a bonding volume, bonding stability, and distribution stability of the organic compound on the surface of the substrate, thereby further improving completeness, stability, and uniformity of the monomolecular film layer formed by the tail group contained in the organic compound in this embodiment of this application on the surface of the substrate. In addition, the head groups can further adjust polarity of the organic compound in this embodiment of this application, thereby further exerting a function of forming the hole transport layer by the organic compound in this embodiment of this application, for example, further enhancement of photoelectric performance such as the open-circuit voltage and photoelectric conversion of the perovskite solar cell. The polarity of the organic compound refers to a dipole moment of the organic compound in this embodiment of this application.

In an embodiment, when the head group contains -Si(ORₐ)₃, Rₐ in the - Si(ORₐ)₃, namely the foregoing first alkyl group, may represent a C1-C5 alkyl group. In an exemplary embodiment, Rₐ may represent a methyl group, an ethyl group, a propyl group, a butyl group, a pentyl group, or the like. Therefore, in an embodiment, when the head group contains -Si(ORₐ)₃, -Si(ORₐ)₃ may represent -Si(OCH₃)₃, -Si(OC2H₅)₃, -Si(OC₃H₇)₃, -Si(OC₄H₉)₃, -Si(OC₅H₁₁)₃, or the like.

In an embodiment, when the head group contains a salt of any one of -SO₃H, -PO(OH)₂, and -COOH, in an exemplary embodiment, the salt of -SO₃H may include at least one of an alkali metal salt, an ammonium salt, a pyridine salt, and a piperazine salt of -SO₃H. The ammonium salt of -SO₃H may include, but not limited to, -SO₃⁻NR₄⁺, where R represents hydrogen or a C1-C5 carbon chain; and the alkali metal salt of-SO₃H may be at least one of -SO₃⁻Li⁺, -SO₃⁻Na⁺, and -SO₃⁻K⁺.

In an exemplary embodiment, the salt of -PO(OH)₂ may include at least one of an alkali metal salt, an ammonium salt, a pyridine salt, and a piperazine salt of-PO(OH)₂. The ammonium salt of -SO₃H may include, but not limited to, at least one of -PO₃²⁻(NR₄⁺)₂ and -PO₂(OH)⁻(NR₄)⁺, where R represents hydrogen or a C1-C5 carbon chain; and the alkali metal salt of -PO(OH)₂ may include at least one of PO₃²⁻(Li⁺)₂,-PO₂(OH)⁻Li⁺, -PO₃²⁻(Na⁺)₂, -PO₂(OH)⁻Na⁺, -PO₃²⁻(K⁺)₂, and -PO₂(OH)⁻K⁺.

In an exemplary embodiment, the salt of -COOH may include at least one of an alkali metal salt, an ammonium salt, a pyridine salt, and a piperazine salt of-COOH. The ammonium salt of -COOH may include, but not limited to, -COO⁻NR₄⁺, where R represents hydrogen or a C1-C5 carbon chain; and the alkali metal salt of-COOH may include at least one of -COO⁻Li⁺, -COO⁻Na⁺, and -COO⁻K⁺.

All the foregoing head groups have good hydrophilicity, which can further enhance a bonding volume, bonding strength, and distribution uniformity of the organic compound in this embodiment of this application on the surface of the substrate such as the conductive substrate, thereby further improving completeness and stability of the monomolecular film layer formed by the tail group of the organic compound in this embodiment of this application. In addition, these head groups can further adjust polarity of the organic compound in this embodiment of this application together with groups such as the tail group and the carbon chain and further improve match between the work function of the electrode, particularly the conductive oxide electrode, and an energy level of a perovskite layer interface, to reduce energy loss and increase the open-circuit voltage of the perovskite cell, thereby significantly enhancing photoelectric conversion efficiency of the perovskite solar cell.

In an embodiment, a tail group contained in the organic compound in the foregoing embodiments may include at least one group in substituted or unsubstituted carbazole, substituted or unsubstituted cyclopentadithiophene, substituted or unsubstituted benzodithiophene, substituted or unsubstituted pyrrolodithiophene, substituted or unsubstituted diphenylamine, and substituted or unsubstituted triphenylamine.

These tail groups represent aromatic groups or aromatic heterocyclic groups, have relatively high hydrophobicity, form a hydrophobic end, and have a π-π interaction effect, which enhances self-assembly of the organic compound in this embodiment of this application into a monomolecular layer through the foregoing types of tail groups. In addition, these groups can adjust polarity of the organic compound in this embodiment of this application together with the head groups as well as the carbon chain, which further increases a bandwidth of the organic compound in this embodiment of this application, enhances extraction of carriers, improves match between the work function of the electrode, particularly the conductive oxide electrode, and the energy level of the perovskite layer interface, reduces energy loss, and increases the open-circuit voltage of the perovskite cell, thereby significantly increasing the photoelectric conversion efficiency of the perovskite solar cell.

When the tail group contained in the organic compound in this embodiment of this application contains a carbazole group, the carbazole group may be expressed as the following group A:

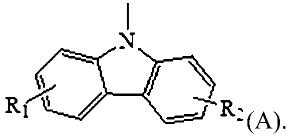

In an embodiment, when the tail group contained in the organic compound in this embodiment of this application contains a cyclopentadithiophene group, the cyclopentadithiophene group may be expressed as the following group B:

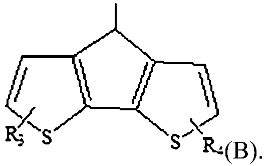

In an embodiment, when the tail group contained in the organic compound in this embodiment of this application contains a benzodithiophene group, the benzodithiophene group may be expressed as the following group C:

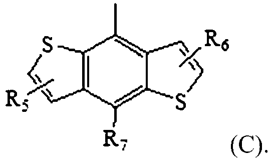

In an embodiment, when the tail group contained in the organic compound in this embodiment of this application contains a pyrrolodithiophene group, the pyrrolodithiophene group may be expressed as the following group D:

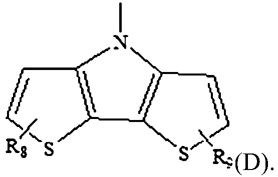

In an embodiment, when the tail group contained in the organic compound in this embodiment of this application contains a diphenylamine group, the diphenylamine group may be expressed as the following group E:

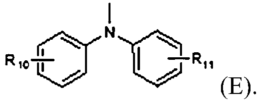

In an embodiment, when the tail group contained in the organic compound in this embodiment of this application contains a triphenylamine group, the triphenylamine group may be expressed as the following group F:

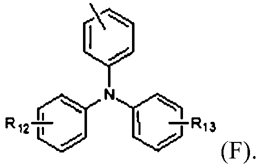

Groups such as R₁, R₂, R₁₀, R₁₁, R₁₂, and R₁₃ in the foregoing groups A, E, and F may be at substitutable positions on a benzene ring, and groups such as R₃, R₄, R₅, R₆, R₈, and R₉ in the foregoing groups B to D may be at substitutable positions of thiophene. In addition, groups of R₁, R₂, R₃, R₄, R₅, R₆, R₇, R₈, R₉, R₁₀, R₁₁, R₁₂, and R₁₃ may independently include any one of hydrogen, halogen, -O-R_{b}, a nitrogen-containing group, -OH, -NHCOR_{c}, -OCOR_{d}, -CH₂COOH, Rₑ, a phenyl group, halogen-substituted R_{f}, a halogen-substituted phenyl group, nitrogen-containing group-substituted R_{g}, and a nitrogen-containing group-substituted phenyl group. R_{b}, R_{c}, R_{d}, Rₑ, R_{f}, and R_{g} independently may represent an alkyl group, denoted herein as a second alkyl group. In an embodiment, the second alkyl group may represent a C1-C5 alkyl group; and in an exemplary embodiment, the second alkyl group may represent a methyl group, an ethyl group, a propyl group, a butyl group, a pentyl group, or the like. Halogen may represent F, Cl, Br, I, or the like.

In an embodiment, when at least one group in R₁, R₂, R₃, R₄, R₅, R₆, R₇, R₈, R₉, R₁₀, R₁₁, R₁₂, and R₁₃ represents the nitrogen-containing group, the nitrogen-containing group may include any one of -N(Rₕ)2, -NHRi, -NH2, a trimethylamino group, a triethylamino group, and a tripropylamino group, where Rₕ and Rᵢ independently may represent an alkyl group, denoted herein as a third alkyl group. In an embodiment, the third alkyl group may represent a C1-C5 alkyl group; and in an exemplary embodiment, the third alkyl group may represent a methyl group, an ethyl group, a propyl group, a butyl group, a pentyl group, or the like.

The substituted or unsubstituted tail groups in the foregoing embodiments all have relatively high hydrophobicity and π-π interaction, which can further enhance self-assembly of the organic compound in the foregoing embodiments into the monomolecular layer through these tail groups, widen a bandgap of the organic compound in the foregoing embodiments, and enhance extraction of carriers. In addition, these substituted or unsubstituted tail groups further adjust polarity of the organic compound in this embodiment of this application together with the foregoing head groups. When the organic compound in this embodiment of this application is used as a hole transport material, match between a work function of an electrode, particularly a conductive oxide electrode, and an energy level of a perovskite layer interface can be further improved, and energy loss and the open-circuit voltage of the perovskite cell are reduced, thereby significantly enhancing photoelectric conversion efficiency of the perovskite solar cell. When the foregoing tail groups include substituents denoted as R₁, R₂, R₃, R₄, R₅, R₆, R₇, R₈, R₉, R₁₀, R₁₁, R₁₂, and R₁₃ (that is, R₁ to R₁₃ represent non-hydrogen atoms), these substituents can further adjust polarity of the organic compound in this embodiment of this application, to further improve match between the work function of the electrode, particularly the conductive oxide electrode, and an energy level of a perovskite layer interface, reduce energy loss and increase the open-circuit voltage of the perovskite cell, thereby significantly enhancing photoelectric conversion efficiency of the perovskite solar cell.

Because the carbon chain contained in the organic compound in the foregoing embodiments connects the foregoing head groups to the tail group, it should be understood that the head groups and the tail group are not connected to the same atom on the carbon chain. Because the number of head groups contained in the organic compound in this embodiment of this application is at least two, in an embodiment, there may be one or more carbon chains. When there is one carbon chain, the at least two head groups are all connected to the carbon chain; and when there are at least two carbon chains, one carbon chain may be connected to one head group, and another head group is connected to another carbon chain. In other embodiments, the head group is connected to one end of the carbon chain, and the tail group is connected to another end of the carbon chain. In this way, connecting the head groups and the tail group to two ends of the same or different carbon chains can effectively reduce steric hindrance of the organic compound in this embodiment of this application and enhance self-assembly of the tail group into the monomolecular layer. In addition, the carbon chain has good flexibility, which can adjust and improve stacking orientation of the tail group and the head groups and increase stability of the organic compound molecule in this embodiment of this application.

In an embodiment, the carbon chain includes at least one of an alkyl chain and a heteroatom-containing alkyl chain. These carbon chains such as the alkyl chain and the heteroatom-containing alkyl chain, particularly the heteroatom-containing alkyl chain, adjust polarity of the organic compound in this embodiment of this application together with the head groups and the foregoing tail group, which can further improve the match between the work function of the electrode, particularly the conductive oxide electrode, and the energy level of the perovskite layer interface, reduce energy loss, and increase the open-circuit voltage of the perovskite cell, thereby significantly increasing the photoelectric conversion efficiency of the perovskite solar cell.

In an embodiment, when the carbon chain contains the alkyl chain or the heteroatom-containing alkyl chain, the number of carbon atoms in the alkyl chain ranges from C1 to C10. The alkyl chain may be a straight-chain alkyl group; and when the number of carbon atoms is 3 or more than 3, the alkyl chain may alternatively be a branched-chain alkyl group. In an exemplary embodiment, the alkyl chain may be a propyl group, a butyl group, a pentyl group, a hexyl group, a heptyl group, an octyl group, a nonyl group, a decyl group, or the like, where groups such as a propyl group, a butyl group, a pentyl group, a hexyl group, a heptyl group, an octyl group, a nonyl group, and a decyl group may be straight-chain alkyl groups or branched-chain alkyl groups. For example, when the carbon chain is the hexyl group, the hexyl group may include, but not limited to, -CH₂CH(CH₂CH₂)₂-.

When the carbon chain is the heptyl group, the heptyl group may include, but not limited to,

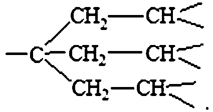

When the carbon chain is the nonyl group, the nonyl group may include, but not limited to,

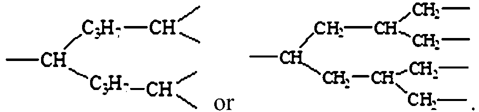

In an embodiment, when the alkyl chain included in the carbon chain contains a heteroatom, the heteroatom may include at least one of O, S, N, B, and Si.

These heteroatom-containing alkyl chains can adjust polarity of the organic compound in this embodiment of this application, which can further improve the match between the work function of the electrode, particularly the conductive oxide electrode, and the energy level of the perovskite layer interface, and the open-circuit voltage of the perovskite cell, and reduce energy loss, thereby significantly increasing the photoelectric conversion efficiency of the perovskite solar cell.

Based on the organic compound in the foregoing embodiments, specifically types of the head groups, carbon chain, and tail group in the foregoing embodiments, the organic compound in this embodiment of this application may include at least one of the following compounds and/or salts of the compounds: 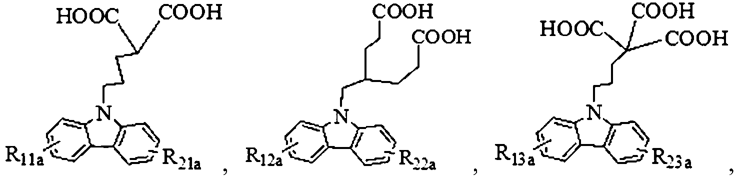 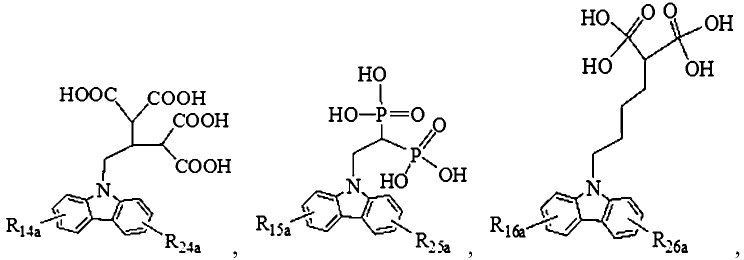 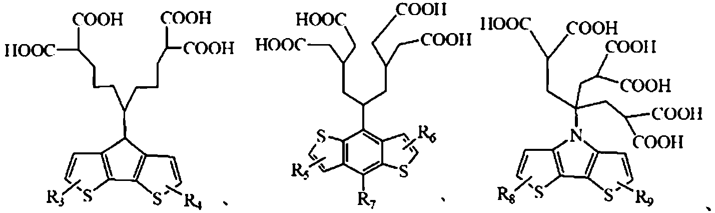 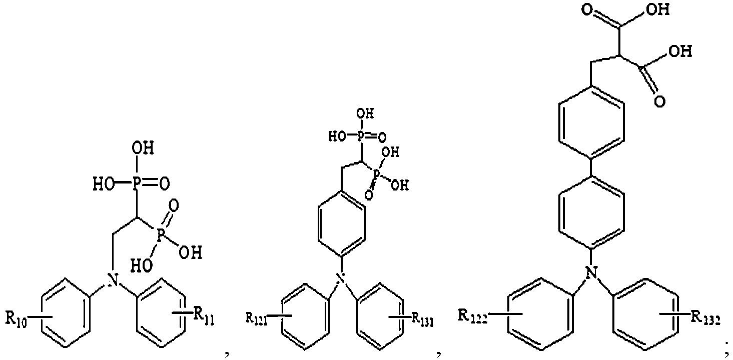 where R₁₁ₐ, R₁₂ₐ, R₁₃ₐ, R₁₄ₐ, R₁₅ₐ, and R₁₆ₐ contained in the organic compound denoted as the foregoing specific chemical formulas independently represent R₁ described above, R₂₁ₐ, R₂₂ₐ, R₂₃ₐ, R₂₄ₐ, R₂₅ₐ, and R₂₆ₐ independently represent R₂ described above, R₁₂₁ and R₁₂₂ independently represent R₁₂, and R₁₃₁ and R₁₃₂ independently represent R₁₃.

The organic compound denoted as the foregoing specific chemical formula contains a plurality of head groups, which can enhance bonding strength and bonding volume of the organic compound for the substrate and improve completeness of the formed monomolecular film layer. In view of this, the head groups, carbon chain, and tail group are selected and designed in terms of assembly, so that the organic compound denoted as the specific chemical formula has proper polarity, which further improves the match between the work function of the electrode, particularly the conductive oxide electrode, and the energy level of the perovskite layer interface, and reduces the energy loss and the open-circuit voltage of the perovskite cell, thereby significantly enhancing the photoelectric conversion efficiency of the perovskite solar cell.

### Preparation Method of Organic Compound

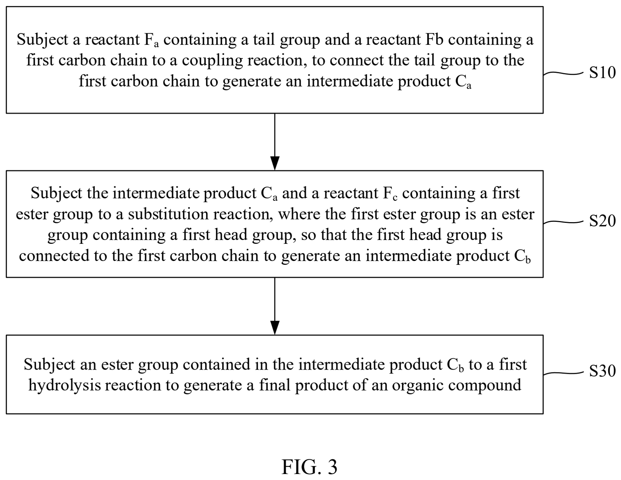

According to a second aspect, an embodiment of this application provides a preparation method of the organic compound in the foregoing embodiment of this application. In some embodiments of this application. FIG. 3 shows a process flow of the preparation method of the organic compound in this embodiment of this application. The preparation method of the organic compound may include the following steps:
S10. Subject a reactant Fₐ containing a tail group and a reactant F_{b} containing a first carbon chain to a coupling reaction, to connect the tail group to the first carbon chain to generate an intermediate product Ca.
S20. Subject the intermediate product Ca and a reactant F_{c} containing a first ester group to a substitution reaction, where the first ester group is an ester group containing a first head group, so that the first head group is connected to the first carbon chain to generate an intermediate product C_{b}.
S30. Subject an ester group contained in the intermediate product C_{b} to a first hydrolysis reaction to generate a final product of an organic compound.

In the preparation method of the organic compound in this embodiment of this application, the two-step reactions of the coupling reaction and the substitution reaction are sequentially performed, so that the tail group and the first head group are connected to the first carbon chain. Through the first hydrolysis reaction in step S30, the ester group undergoes the first hydrolysis reaction, so that the first head group constitutes a hydrophilic end of the organic compound. Because the preparation method of the organic compound in this embodiment of this application is intended for preparation of the organic compound in the foregoing embodiments of this application, the reactant F_{c} may contain at least one or more first head groups, and the final product of the organic compound contains at least two first head groups. The term "at least two" in this specification of the embodiments of this application means "two or more than two".

Therefore, in the preparation method of the organic compound in this embodiment of this application, the tail group and at least two first head groups can be effectively connected to the first carbon chain, to form the organic compound in the foregoing embodiments of this application. In addition, the number of first head groups that are connected to the first carbon chain and that are contained in the reactant F_{c} can be controlled, so that the number of first head groups contained in the final product of the organic compound is adjusted to at least two, to endow the prepared organic compound with features of the organic compound in the foregoing embodiments of this application. For example, the prepared organic compound has high bonding strength and large bonding volume for the surface of the substrate such as the conductive substrate and is uniformly distributed, which improves completeness and stability of the monomolecular film layer formed by the tail group contained in the organic compound in this embodiment of this application on the surface of the substrate and improves uniformity of the monomolecular film layer. In addition, the first head groups can further adjust the polarity of the organic compound together with the tail group and the first carbon chain, to fully exert effects of forming the hole transport layer by the organic compound, for example effective improvement of the match between the work function of the electrode, particularly the conductive oxide electrode, and the energy level of the perovskite layer interface, and the open-circuit voltage of the perovskite cell, and a decrease in the energy loss, thereby significantly increasing the photoelectric conversion efficiency of the perovskite solar cell. In addition, the preparation method of the organic compound in this embodiment of this application features easily controllable reaction conditions, few byproducts, and a high yield of the final product.

### Step S10:

The reactants Fₐ and F_{b} are subjected to the coupling reaction in step S10, so that the tail group contained in the reactant Fₐ is connected to the first carbon chain. Therefore, in addition to the tail group contained in the reactant Fₐ and the first carbon chain contained in the reactant F_{b}, the reactants Fₐ and F_{b} respectively further contain groups that can be subjected to the coupling reaction, to subject the two reactants to the coupling reaction, thereby connecting the tail group to the first carbon chain.

Based on the reactants and their reaction type in step S10, in an embodiment, the reactant Fₐ may include at least one of the following compounds: 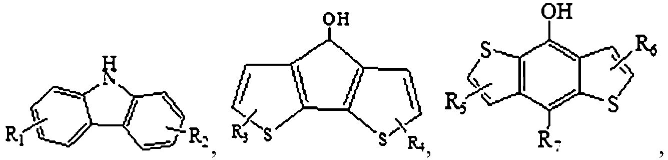 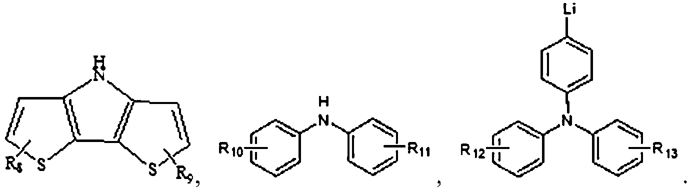 where R₁, R₂, R₃, R₄, R₅, R₆, R₇, R₈, R₉, R₁₀, R₁₁, R₁₂, and R₁₃ respectively correspond to R₁, R₂, R₃, R₄, R₅, R₆, R₇, R₈, R₉, R₁₀, R₁₁, R₁₂, and R₁₃ contained in the organic compound in the foregoing embodiments of this application. For example, in an embodiment, R₁, R₂, R₃, R₄, R₅, R₆, R₇, R₈, R₉, R₁₀, R₁₁, R₁₂, and R₁₃ may independently include any one of hydrogen, halogen, -O-R_{b}, a nitrogen-containing group, -OH, -NHCOR_{c}, -OCOR_{d}, -CH₂COOH, Rₑ, a phenyl group, halogen-substituted R_{f}, a halogen-substituted phenyl group, nitrogen-containing group-substituted R_{g}, and a nitrogen-containing group-substituted phenyl group, where R_{b}, Rₑ, R_{d}, Rₑ, R_{f}, and R_{g} independently represent an alkyl group, denoted herein as a fourth alkyl group. Therefore, the fourth alkyl group may be the same as the foregoing second alkyl group. For example, in an embodiment, the fourth alkyl group may represent a C1-C5 alkyl group; and in an exemplary embodiment, the fourth alkyl group may represent a methyl group, an ethyl group, a propyl group, a butyl group, a pentyl group, or the like.

In an embodiment, the reactant F_{b} may be a compound containing any one of the alkyl chain and the heteroatom-containing alkyl chain, namely the first carbon chain, contained in the organic compound in the foregoing embodiments of this application. For example, the reactant F_{b} may include any one of a haloalkane compound containing any one of the alkyl chain and the heteroatom-containing alkyl chain, a heteroatom-containing haloalkane compound, and a halogenated alkyl acyl halide compound. The halogen element may be F, Cl, Br, I, or the like; and the heteroatom may include at least one of O, S, N, B, and Si.

In an embodiment, an environment of the coupling reaction in step S01 may be an alkaline environment. For example, an alkaline compound is contained. An amount of added alkaline compound should be proper to ensure the normal coupling reaction. For example, a regular amount of alkaline compound in the coupling reaction is added. In addition, the alkaline compound may be inorganic and organic alkalis; and in an embodiment, the inorganic alkali may include at least one of a carbonate, a hydroxide, and the like of an alkali metal. The carbonate may include, but not limited to, potassium carbonate and sodium carbonate. The hydroxide may include, but not limited to, sodium hydroxide and potassium hydroxide. The organic alkali may include at least one of triethylamine, pyridine, imidazole, a methoxide of the alkali metal, an ethoxide of the alkali metal, an n-butoxide of the alkali metal, a t-butoxide of the alkali metal, and a hydride. The methoxide, ethoxide, n-butoxide, and t-butoxide of the alkali metal include, but are not limited to, sodium methoxide, sodium ethoxide, sodium n-butoxide, sodium t-butoxide, and potassium t-butoxide. The hydride may include, but not limited to, sodium hydride.

A solvent of the coupling reaction system may include at least one of N,N-dimethylformamide, toluene, water, 1,2-xylene, chlorobenzene, 1,2-dichlorobenzene, tetrahydrofuran, and ethanol.

Conditions of the coupling reaction system are controlled, which can increase efficiency of the coupling reaction and a yield of the intermediate product Ca. In addition, the reactant Fₐ and the reactant F_{b} may be mixed at a molar ratio in a chemical equation for the coupling reaction of the reactant Fa and the reactant Fb. Certainly, a properly excessive amount of one reactant such as the reactant Fₐ or the reactant F_{b} may be used to ensure complete reaction of the other reactant.

### Step S20:

The reactant F_{c} and the intermediate product Ca are subjected to the substitution reaction in step S20, so that an ester group of the first head group contained in the reactant F_{c}, namely an ester group, is connected to the first carbon chain. To be specific, the first head group is connected to the first carbon chain. Therefore, in addition to the ester group of the first head group, namely the ester group, contained in the reactant F_{c}, the reactant F_{c} and the intermediate product Ca respectively further contain groups that can be subjected to the substitution reaction, so that the two reactants can be subjected to the substitution reaction, to connect the first head group to the first carbon chain. Because the purpose is to prepare the organic compound in the foregoing embodiments of this application, an atom, connected to the first carbon chain, of the first head group may be the same as or different from an atom, connected to the first carbon chain, of the tail group.

Based on the reactants and their reaction type in step S20, in an embodiment, the reactant F_{c} may include at least one compound containing an ester group of -SO₃H, an ester group of -PO(OH)₂, an ester group of -COOH, or -Si(ORₐ). There may be one or more ester groups. Rₐ represents an alkyl group, denoted herein as a fifth alkyl group. Therefore, the fifth alkyl group may be the same as the foregoing first alkyl group. For example, in an embodiment, the fifth alkyl group may be a C1-C5 alkyl group; and in an exemplary embodiment, the fifth alkyl group may be a methyl group, an ethyl group, a propyl group, a butyl group, a pentyl group, or the like.

In an embodiment, the substitution reaction in step S02 may be conducted in the presence of an alkaline compound. In an embodiment, the alkaline compound may be at least one of NaH and the hydroxide, methoxide, ethoxide, n-butoxide, and t-butoxide of the alkali metal. For example, in the presence of NaH, a methylene group contained in the reactant F_{c}, for example a methylene group of diethyl malonate, is likely to form a carbanion, to be subjected to the substitution reaction with the intermediate product Ca to generate the intermediate product C_{b}.

A solvent of the substitution reaction system is a polar solvent; and in an exemplary embodiment, the polar solvent may include at least one of N,N-dimethylformamide, tetrahydrofuran, dimethyl sulfoxide, anhydrous ethanol, anhydrous diethyl ether, and toluene.

Conditions of the substitution reaction system are controlled, which can increase efficiency of the substitution reaction and a yield of the intermediate product C_{b}. In addition, the reactant F_{c} and the intermediate product Ca may be mixed at a molar ratio of a chemical equation for the substitution reaction of the reactant Fc and the intermediate product Ca. Certainly, a properly excessive amount of one reactant such as the reactant F_{c} or the intermediate product Ca may be used to ensure complete reaction of the other reactant.

### Step S30:

The first hydrolysis reaction in step S30 is intended for hydrolysis of the ester group contained in the intermediate product C_{b}, to generate the first head group, so that the final product of the organic compound contains at least two first head groups with hydrophilicity.

Based on the type and purpose of the first hydrolysis reaction in step S30, in an embodiment, the first hydrolysis reaction may be carried out first in a basic solution and then in an acidic environment, so that the ester group contained in the intermediate product C_{b} is hydrolyzed to generate the first head group. An alkali in the basic solution may be an alkali capable of hydrolyzing the ester group, for example a hydroxide of an alkali metal, including, but not limited to, sodium hydroxide; and an acid in the acidic environment may be an inorganic acid, including, but not limited to, a hydrochloric acid solution. In other embodiments, the first hydrolysis reaction may involve a hydrolysis catalyst that can improve efficiency of the first hydrolysis reaction; and in an exemplary embodiment, the hydrolysis catalyst may include at least one of trimethylsilyl bromide, trimethylsilyl chloride, and trimethylsilyl iodide. A properly excessive amount of hydrolysis catalyst may be added to minimally ensure occurrence of the first hydrolysis reaction.

A solvent of the first hydrolysis reaction may include, but not limited to, at least one of 1,4-dioxane, water, anhydrous ethanol, toluene, 1,2-xylene, chlorobenzene, 1,2-dichlorobenzene, and tetrahydrofuran.

Conditions of the first hydrolysis reaction system are controlled and optimized, which can increase efficiency of the first hydrolysis reaction and a yield of the final product.

In other embodiments of this application, the organic compound in this embodiment of this application may also be prepared in a preparation method including the following steps:
S40. Subject a reactant Fₐ containing a tail group and a reactant Fₑ containing a second carbon chain and a second ester group to an addition reaction, to connect the tail group to the second carbon chain to generate an intermediate product C_{c}, where the second ester group is an ester group containing a second head group, and the second head group is connected to the second carbon chain.
S50. Subject the second ester group contained in the intermediate product C_{c} to a second hydrolysis reaction to generate a final product of the organic compound.

In the preparation method of the organic compound in this embodiment of this application, the one-step addition reaction is directly carried out, to connect the tail group and the second head group to the second carbon chain. Because the purpose is to prepare the organic compound in the foregoing embodiments of this application, the second ester group contained in the reactant Fₑ is the ester group of the second head group, and the second head group is connected to the second carbon chain. After the second hydrolysis reaction in step S50, the second ester group undergoes the second hydrolysis reaction, so that the second head group constitutes a hydrophilic end of the organic compound. Because the preparation method of the organic compound in this embodiment of this application is intended for preparation of the organic compound in foregoing embodiments of this application, the second head group may be the foregoing first head group, including, for example, any one of -SO₃H, -PO(OH)₂, -COOH, and - Si(ORₐ)₃. Rₐ represents an alkyl group, denoted herein as a sixth alkyl group. Therefore, the sixth alkyl group may be the same as the foregoing first alkyl group. In an embodiment, the sixth alkyl group may be a C1-C5 alkyl group; and in an exemplary embodiment, the sixth alkyl group may be a methyl group, an ethyl group, a propyl group, a butyl group, a pentyl group, or the like. In addition, the second head group should be connected to the second carbon chain of the reactant Fₑ. Therefore, the second ester group may be the same as the foregoing first ester group.

The second carbon chain may be the same as the foregoing first carbon chain, including, for example, any one of the alkyl chain and the heteroatom-containing alkyl chain.

Therefore, in the preparation method of the organic compound in this embodiment of this application, the tail group and the second head group can be effectively connected to the second carbon chain at a time, to form the organic compound in the foregoing embodiments of this application. In addition, a type of the tail group and types of the second head group and the second carbon chain contained in the reactant Fₑ can be selected to adjust performance such as polarity of the final product of the organic compound, so that the final product of the organic compound can be used as the hole transport material, which can further improve the match between the work function of the electrode, particularly the conductive oxide electrode, and the energy level of the perovskite layer interface, and the open-circuit voltage of the perovskite cell, and reduce energy loss, thereby significantly increasing the photoelectric conversion efficiency of the perovskite solar cell. In addition, the preparation method of the organic compound in this embodiment of this application features easily controllable reaction conditions, few byproducts, and a high yield of the final product.

### Step S40:

The reactant Fₐ and the reactant Fₑ are subjected to the addition reaction in step S40, so that a tail group contained in the reactant Fₐ is connected to the second carbon chain. Therefore, in addition to the tail group contained in the reactant Fₐ and the second head group and the second carbon chain contained in the reactant Fₑ, the reactants Fₐ and Fₑ respectively further contain groups that can be subjected to the addition reaction, so that the two reactants can be subjected to the addition reaction, to connect the tail group to the second carbon chain. Because the purpose is to prepare the organic compound in foregoing embodiments of this application, an atom, connected to the second carbon chain, of the second head group may be the same as or different from an atom, connected to the second carbon chain, of the tail group.

Based on the reactants and their reaction type in step S40, in an embodiment, the reactant Fₑ may include any one of a halogenated compound containing an ester group of the second head group, namely the second ester group (including at least one of an ester group of -SO₃H, an ester group of -PO(OH)₂, an ester group of -COOH, and -Si(ORₐ)₃) and the second carbon chain (including, for example, any one of the alkyl chain and the heteroatom-containing alkyl chain), a heteroatom-containing haloalkane compound, and a halogenated alkyl acyl halide compound. Rₐ represents an alkyl group, denoted herein as a sixth alkyl group. Therefore, the sixth alkyl group may be the same as the foregoing first alkyl group. For example, in an embodiment, the sixth alkyl group may represent a C1-C5 alkyl group; and in an exemplary embodiment, the sixth alkyl group may represent a methyl group, an ethyl group, a propyl group, a butyl group, a pentyl group, or the like.

In an exemplary embodiment, the reactant Fₑ includes at least one of the following compounds: 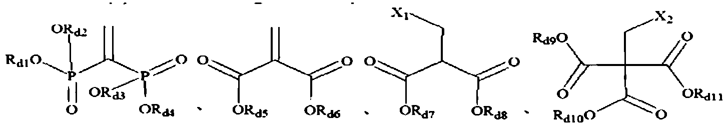 where R_{d1}, R_{d2}, R_{d3}, R_{d4}, R_{d5}, R_{d6}, R_{d7}, R_{d8}, R_{d9}, R_{d10}, and R_{d11} independently represent a C1-C5 alkyl chain, and X₁ and X₂ independently represent a halogen atom.

In an embodiment, an environment of the addition reaction in step S40 may be an environment allowing occurrence of the addition reaction. For example, an alkaline compound is added to the addition reaction. In an embodiment, the alkaline compound may be an inorganic or organic alkaline compound; and in an exemplary embodiment, the inorganic alkaline compound may include, but not limited to, a hydroxide. The organic alkaline compound may include, but not limited to, butyllithium, specifically n-butyllithium.

A solvent of the addition reaction system may include at least one of tetrahydrofuran, anhydrous ethanol, toluene, 1,2-xylene, chlorobenzene, and 1,2-dichlorobenzene.

Conditions of the addition reaction system are controlled, which can increase efficiency of the addition reaction and a yield of the intermediate product C_{c}. In addition, the reactant F_{d} and the reactant Fₑ may be mixed at a molar ratio of a chemical equation for the addition reaction of the reactant F_{d} and the reactant Fₑ. Certainly, a properly excessive amount of one reactant such as the reactant F_{d} or the reactant Fₑ may be used to ensure complete reaction of the other reactant.

### Step S50:

The second hydrolysis reaction in step S50 is intended for hydrolysis of the second ester group contained in the intermediate product C_{c}, to generate the second head group, so that the final product of the organic compound contains the second head group with hydrophilicity.

Based on the type and purpose of the second hydrolysis reaction in step S50, in an embodiment, conditions of the second hydrolysis reaction may be the same as those of the foregoing first hydrolysis reaction system. For example, the hydrolysis catalyst may be involved to improve efficiency of the second hydrolysis reaction. For example, in an embodiment, the hydrolysis catalyst may include at least one of trimethylsilyl bromide, trimethylsilyl chloride, and trimethylsilyl iodide. A properly excessive amount of hydrolysis catalyst may be added to minimally ensure occurrence of the second hydrolysis reaction.

A solvent of the second hydrolysis reaction system is an aprotic solvent; and in an embodiment, the aprotic solvent may also include, but not limited to, at least one of 1,4-dioxane, toluene, 1,2-xylene, chlorobenzene, 1,2-dichlorobenzene, and tetrahydrofuran.

Types of the hydrolysis catalyst and the solvent are controlled and optimized, which can increase efficiency of the second hydrolysis reaction and a yield of the final product.

In a further embodiment, in addition to the foregoing step S30 and/or step S50, the preparation method of the organic compound further includes the following step:
subjecting the final product of the organic compound prepared in step S30 or the final product of the organic compound prepared in step S50 and a soluble salt solution to a reaction, to generate a corresponding salt of the final product of the organic compound.

The amount of soluble salt may be excessive relative to the final product of the organic compound to ensure complete conversion of the final product of the organic compound into the corresponding salt. In addition, the soluble salt solution may be an alkali metal salt solution, an ammonium salt solution, or the like.

### Hole Transport Material

According to a third aspect, an embodiment of this application provides a hole transport material. The hole transport material in this embodiment of this application includes the organic compound in the foregoing embodiments of this application.

Therefore, the hole transport material in this embodiment of this application has high bonding strength for an electrode, and can form a monomolecular layer on a surface of the electrode and also have a wide bandgap, which can effectively improve match between a work function of an electrode, particularly a conductive oxide electrode, and an energy level of a perovskite layer interface, improve extraction of carriers, reduce energy loss, and increase an open-circuit voltage of a perovskite cell, thereby significantly enhancing photoelectric conversion efficiency of a perovskite solar cell.

In an embodiment, the hole transport material in this embodiment of this application may contain only the organic compound in the foregoing embodiments of this application. In an application process of the hole transport material, the organic compound in the foregoing embodiments of this application may individually form a film to form a hole transport layer. Alternatively, a passivation layer may be formed on a surface of a metal oxide hole transport layer to passivate defects on a surface of the metal oxide hole transport layer, thereby improving hole transport performance together with the metal oxide hole transport layer.

Certainly, in some embodiments, the hole transport material in this embodiment of this application may further contain other components such as other materials with a hole transport capability or auxiliary components capable of assisting the organic compound in foregoing embodiments of this application in exerting its function. When the hole transport material in this embodiment of this application contains other components, the other components and the organic compound in foregoing embodiments of this application may be mixed at a certain ratio, specifically based on a mixing principle of improving the foregoing function of the organic compound in foregoing embodiments of this application.

### Perovskite Solar Cell

According to a fourth aspect, an embodiment of this application provides a perovskite solar cell. In some embodiments of this application, a structure of the perovskite solar cell in this embodiment of this application may be a structure shown in FIG. 1, and may minimally include a first electrode, a hole transport layer, a perovskite layer, an electron transport layer, and a second electrode that are sequentially stacked.

The material of the perovskite layer contains the organic compound in foregoing embodiments of this application or the hole transport material in foregoing embodiments of this application.

In this way, the material contained in the hole transport layer in this embodiment of this application has a wide bandgap, which can effectively improve match between a work function of a first electrode and an energy level of a perovskite layer interface, significantly improve extraction of carriers, reduce energy loss, and increase an open-circuit voltage of a perovskite cell, thereby significantly enhancing photoelectric conversion efficiency of a perovskite solar cell.

In an embodiment, a thickness of the hole transport layer may range from 0.1 nm to 10 nm, or further optionally 0.5 nm to 10 nm, 0.1 nm to 5 nm, 1 nm to 5 nm, 3 nm to 5 nm, or 2 nm to 4 nm; and in an exemplary embodiment, the thickness of the perovskite layer may be a typical but unrestrictive thickness such as 0.1 nm, 0.5 nm, 1 nm, 1.5 nm, 2 nm, 3 nm, 4.5 nm, 5 nm, 8 nm, or 10 nm.

The thickness of the hole transport layer is controlled and optimized, which can help exert functions of the hole transport layer, for example, enhancement of the match between the work function of the first electrode and the energy level of the perovskite layer interface, an increase in extraction of carriers, a decrease in energy loss, and an increase in the open-circuit voltage of the perovskite cell, thereby significantly enhancing the photoelectric conversion efficiency of the perovskite solar cell.

Based on the foregoing hole transport layer, in an embodiment, the first electrode is a conductive oxide electrode; and in this case, the hole transport layer is stacked between the first electrode and the perovskite layer. The hole transport layer is stacked and bonded with the conductive oxide electrode. On the one hand, chemical bond adsorption of the organic compound in the foregoing embodiments of this application and the conductive oxide electrode layer can be improved, specifically the chemical bond adsorption of the head group of the organic compound in foregoing embodiments of this application and the conductive oxide substrate, thereby further improving bonding strength of the hole transport layer for the conductive oxide electrode and enhancing completeness of a monomolecular film layer formed by the organic compound on a surface of the conductive oxide electrode. On the other hand, energy level match between the conductive oxide electrode and the perovskite layer is improved, thereby increasing photoelectric performance such as the open-circuit voltage and photoelectric conversion efficiency of the perovskite solar cell.

In an embodiment, a material of the first electrode or the conductive oxide electrode may include, but not limited to, at least one of a transparent material of a fluorine-doped tin oxide (FTO), indium tin oxide (ITO), a transparent conductive material of an aluminum-doped zinc oxide (AZO), a transparent conductive material of a boron-doped zinc oxide (BZO), and a transparent conductive material of an indium-doped zinc oxide (IZO). These conductive oxide materials form a conductive oxide electrode layer that is highly transparent and that has high bonding strength when stacked on the hole transport layer. Under an action of the hole transport layer, the work function of the conductive oxide electrode can be effectively decreased to become closer to a quasi-Fermi level of the perovskite layer, which increases the open-circuit voltage of the perovskite cell as well as the match between the work function of the conductive oxide electrode and the energy level of the perovskite layer interface, improves extraction of carriers, and reduces energy loss, thereby significantly enhancing photoelectric conversion efficiency of the perovskite solar cell.

In an embodiment, a chemical formula of perovskite contained in the perovskite layer satisfies ABX₃ or A₂CDX₆. A represents an inorganic or organic cation or a mixture of organic and inorganic cations, and may represent at least one of a methylammonium ion (MA⁺, CH₃NH₃⁺), a formamidinium ion (FA⁺, HC(NH₂)₂⁺), and Cs⁺; B represents an inorganic or organic cation or a mixture of organic and inorganic cations, and may represent at least one of Pb²⁺ and Sn²⁺; C represents an inorganic or organic cation or a mixture of organic and inorganic cations, which is commonly Ag⁺; D represents an inorganic or organic cation or a mixture of organic and inorganic cations, and may represent at least one of a bismuth cation Bi³⁺, an antimony cation Sb³⁺, and an indium cation In³⁺; and X represents an inorganic or organic anion or a mixture of organic and inorganic anions, and may represent at least one of Br⁻ and I⁻. The perovskite of this material has high solar energy absorption and an energy level that highly matches the work function of the first electrode, particularly the foregoing conductive oxide electrode, under the action of the hole transport layer, and a bandgap of the perovskite layer can reach 1.20-2.30 eV.

In an embodiment, a thickness of the perovskite layer may range from 200 nm to 1000 nm, or further optionally from 300 nm to 800 nm; and in an exemplary embodiment, the thickness of the perovskite layer may be a typical but unrestrictive thickness such as 20 nm, 100 nm, 200 nm, 300 nm, 400 nm, 500 nm, 600 nm, 700 nm, 800 nm, 900 nm, or 1000 nm.

In an embodiment, a material of the electron transport layer includes at least one of an electron transport material, a derivative of the electron transport material, a dopant of the electron transport material, and a passivated product of the electron transport material; and the electron transport material includes at least one of methyl[6,6]-phenyl-C₆₁-butyrate (PC₆₁BM), methyl[6,6]-phenyl-C₇₁-butyrate (PC₇₁BM), fullerene C₆₀ (C₆₀), fullerene C₇₀ (C₇₀), tin dioxide (SnO₂), and zinc oxide (ZnO).

These materials can effectively increase electron extraction and transport of the electron transport layer and synergize with functional layers such as the hole transport layer, thereby further improving the photoelectric conversion efficiency of the perovskite solar cell.

In an embodiment, a material of the second electrode includes an organic or inorganic conductive material or a mixture of organic and inorganic conductive materials; and in an exemplary embodiment, the material includes, but is not limited to, Ag, Cu, C, Au, Al, FTO, ITO, AZO, BZO, and IZO. For example, when the foregoing first electrode is the conductive oxide electrode, a metal electrode may be selected as the second electrode.

In some embodiments, the perovskite solar cells in the foregoing embodiments may further include a transparent substrate; and in an exemplary embodiment, the first electrode of the perovskite solar cell is bonded onto a surface of the transparent substrate. In an embodiment, a material of the transparent substrate is glass or another transparent material; and another transparent material may include, but not limited to, transparent resin.

In some embodiments, the perovskite solar cell in the foregoing embodiments may further include a passivation layer, where the passivation layer is stacked between the electron transport layer and the second electrode. In an exemplary embodiment, a material of the passivation layer may be a hole-blocking material, including, but not limited to, 2,9-dimethyl-4,7-diphenyl-1,10-phenanthroline (BCP), nano-ZnO, and SnO.

### Preparation Method of Perovskite Solar Cell

According to a fifth aspect, an embodiment of this application further provides a preparation method of a perovskite solar cell. In some embodiments, the preparation method of the perovskite solar cell in this embodiment of this application includes the following steps:
S60. Provide a first electrode.
S70. Prepare a slurry including an organic compound or a hole transport material, and subject the slurry to film-forming processing on a surface of the first electrode to form a hole transport layer.
S80. Form a perovskite layer on a surface, facing away from the first electrode, of the hole transport layer.
S90. Form an electron transport layer on a surface, facing away from the hole transport layer, of the perovskite layer.
S100. Form a second electrode on a surface, facing away from the perovskite layer, of the electron transport layer.

### Step S60:

The first electrode in step S60 is the first electrode contained in the perovskite solar cell in the foregoing embodiments of this application; in an embodiment, the first electrode may be a conductive oxide electrode; and in an exemplary embodiment, when the first electrode is the conductive oxide electrode, the conductive oxide electrode may use the foregoing conductive materials such as a transparent material of the fluorine-doped tin oxide (FTO), indium tin oxide (ITO), a transparent conductive material of an aluminum-doped zinc oxide (AZO), a transparent conductive material of a boron-doped zinc oxide (BZO), and a transparent conductive material of an indium-doped zinc oxide (IZO) or conductive substrate. The conductive oxide electrode is used as the first electrode, and therefore, the hole transport layer formed in step S07 is directly stacked onto and bonded with the conductive oxide electrode, and chemical bonds between the organic compound, contained in the slurry in step S07, in the foregoing embodiments of this application and the conductive oxide electrode layer are effectively formed, which further improves bonding strength of the hole transport layer for the conductive oxide electrode, enhances completeness of a monomolecular film layer formed by the organic compound on a surface of the conductive oxide electrode, and improves energy level match between the conductive oxide electrode and the perovskite layer, thereby enhancing photoelectric performance such as the open-circuit voltage and photoelectric conversion efficiency of the perovskite solar cell.

In an embodiment, the method may include etching and cleaning the first electrode to remove surface contaminants or improve surface properties of the first electrode, enhancing bonding strength of the hole transport layer on the surface of the first electrode and fully exerting functions such as adjusting a work function of the first electrode by the hole transport layer.

### Step S70:

The organic compound in step S70 is the organic compound in the foregoing embodiments of this application, and the hole transport material is the hole transport material in the foregoing embodiments of this application. In this case, because the slurry contains the organic compound in this embodiment of this application, the head group contained in the organic compound has good hydrophilicity, so that the organic compound in this embodiment of this application can be effectively bonded to a surface of the first electrode such as the conductive oxide. With the good hydrophobicity, the tail groups contained in the organic compound in this embodiment of this application can be self-assembled into a monomolecular film layer, to fully exert the foregoing functions of the organic compound in this embodiment of this application, thereby enhancing the photoelectric conversion efficiency of the perovskite solar cell in this embodiment of this application. In addition, because the formed hole transport layer has high bonding strength for the surface of the first electrode such as the conductive oxide, when a perovskite layer is formed on a surface of the hole transport layer, the hole transport layer can resist erosion of a perovskite precursor solution, to ensure stability of the hole transport layer, that is, completeness and stability of a monomolecular layer formed on the surface of the first electrode such as the conductive oxide.

In an embodiment, a concentration of the slurry may be controlled to range from 0.1 mg/mL to 10 mg/mL. This concentration refers to a concentration of the organic compound in this embodiment of this application or the hole transport material in the foregoing embodiments of this application, which may also be understood as a solute in the slurry. The concentration of the slurry is controlled to fall within this range, so that the head group of the organic compound in this embodiment of this application is fully bonded to the surface of the first electrode such as the conductive oxide and the tail groups are fully self-assembled into a complete monomolecular film layer, thereby improving film quality of the hole transport layer.

In an embodiment, a solvent of the slurry may include, but not limited to, solvents such as methanol, isopropanol, ethanol, and chlorobenzene; and these solvents can effectively dissolve the organic compound in this embodiment of this application or the hole transport material in the foregoing embodiments of this application, to form a uniform slurry, so that a hydrophilic head group of the organic compound in this embodiment of this application is bonded to the surface of the first electrode such as the conductive oxide and the hydrophobic tail group can be self-assembled into the monomolecular film layer.

In an embodiment, through conditions of the film-forming processing, a thickness of the formed hole transport layer can be controlled to range from 0.1nm to 10 nm.

In an embodiment, the film-forming processing includes a combination of one or more of spin coating, spray coating, blade coating, slot-die coating, roll-to-roll printing, and soaking of the surface of the first electrode in the slurry; and any available method for forming the hole transport layer on the surface of the first electrode by using the slurry falls within the scope disclosed in this specification of the embodiments of this application.

### Step S80 and Step S90:

Methods of forming the perovskite layer in step S80, the electron transport layer in step S90, and the second electrode in step 100 may be performed according to features of the materials. For example, appropriate methods are selected to form the perovskite, the electron transport layer, and the second electrode respectively based on features of the perovskite material contained in the perovskite in the foregoing embodiments of this application, the electron transport material, and the material of the second electrode.

In some embodiments, when the perovskite in this application contains a passivation layer (or a hole-blocking layer), between step S90 and step S100, that is, between the step of forming the electron transport layer and the step of forming the second electrode, the method further includes: forming the passivation layer on a surface, facing away from the perovskite layer, of the electron transport layer, and then forming the second electrode on a surface, facing away from the electron transport layer, of the passivation layer. A method of forming the passivation layer is also selected appropriately based on characteristics of a passivation material.

### Electric Apparatus

According to a sixth aspect, an embodiment of this application further provides an electric apparatus. The electric apparatus includes the perovskite solar cell in the foregoing embodiments of this application. The perovskite solar cell serves as a power supply to supply power to the foregoing electric apparatus; or the perovskite solar cell may serve as an energy storage unit of the electric apparatus. Exemplarily, the electric apparatus may be a lighting element, a display element, a vehicle, or the like.

Exemplarily, the electric apparatus may include, but not limited to, a mobile device (for example, a mobile phone, a tablet computer, or a notebook computer), an electric vehicle (for example, a battery electric vehicle, a hybrid electric vehicle, a plug-in hybrid electric vehicle, an electric bicycle, an electric scooter, an electric golf vehicle, or an electric truck), an electric train, a ship, a satellite, an energy storage system, a lighting element, a display element, or the like.

### Examples

The following describes examples of this application. The examples described below are exemplary and only intended to explain this application, and shall not be construed as a limitation on this application. If no specific techniques or conditions are specified in an example, techniques or conditions described in literature in the field or product instructions are followed. The reagents or instruments used are all conventional products commercially available if no manufacturer is indicated.

### 1. Examples of Organic Compound and Preparation Method Thereof:

### Example A1:

This example provides an organic compound and a preparation method thereof. The organic compound in this example is denoted as the following chemical formula 5.

A preparation method of the organic compound denoted as chemical formula 5 includes the following steps:
S1. 1.97g of compound 1, 2.02g of triethylamine, and 1.56g of acetyl chloride were added to a three-necked flask and dissolved in 50 mL of dichloromethane; after being stirred for 20 hours, the mixture was added into 50 mL of an aqueous saturated ammonium chloride solution and extracted three times with 50 mL of dichloromethane; and a yellow oily substance obtained after removal of the solvent was separated by silica gel column chromatography to obtain compound 2 (2.75g, 98%), with ¹H NMR (400 MHz, DMSO-*d*6) *δ* 11.66 (s, 1H), 10.11 (s, 2H), 7.82-7.80 (m, 4H), 7.68 (d, *J =* 7.8, 2H), 2.06 (s, 6H) measured; and a chemical equation of compound 1 and acetyl chloride is as follows: 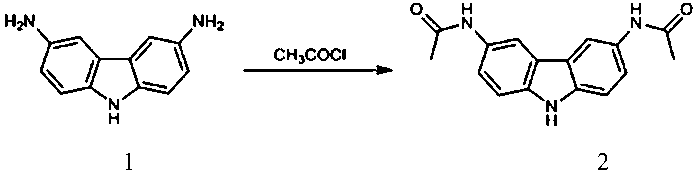
S2. 2.75g of compound 2, 1.85g of 1,2-dibromoethane, and 1.52g of potassium carbonate were dissolved in 20 mL of N,N-dimethylformamide, the mixture was heated at 85°C for 20 hours, then added into 50 mL of an aqueous saturated sodium chloride solution and extracted three times with 50 mL of ethyl acetate; and a yellow oily liquid obtained after removal of the solvent was separated by silica gel column chromatography to obtain compound 3 (2.54g, 67%), with ¹H NMR (400 MHz, CDCl3) *δ* 10.11 (s, 2H), 7.89-7.61 (m, 6H), 4.38-4.32 (m, 2H), 3.59-3.55 (m, 2H), 2.06 (s, 6H) measured; and a chemical equation of compound 2 and 1,2-dibromoethane is as follows: 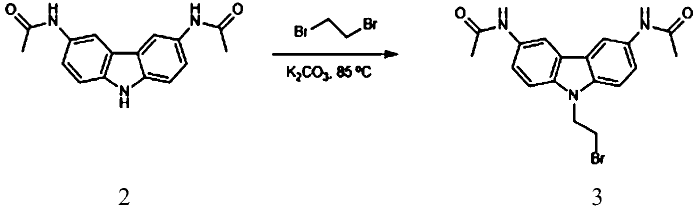
S3. 0.36g of NaH was added to a three-necked flask containing 20 mL of N,N-dimethylformamide, 1.04g of diethyl malonate was slowly added dropwise, and after 0.5 hours of reaction, a solution of 2.54g of compound 3 in N,N-dimethylformamide (20 mL) was slowly added dropwise into the three-necked flask; after reacting overnight at 50°C, the mixture was added into 50 mL of an aqueous saturated sodium chloride solution and extracted three times with 50 mL of ethyl acetate; and a yellow oily liquid obtained after removal of the solvent was separated by silica gel column chromatography to obtain compound 4 (2.05g, 67%), with ¹H NMR (400 MHz, CDCl3) *δ* 10.11 (s, 2H), 7.89-7.60 (m, 6H), 4.18-4.10 (m, 6H), 3.35-3.27 (m, 1H), 2.33-2.28 (m, 2H), 2.06 (s, 6H), 1.23-1.19 (m, 6H) measured; and a chemical equation of compound 3 and diethyl malonate is as follows: 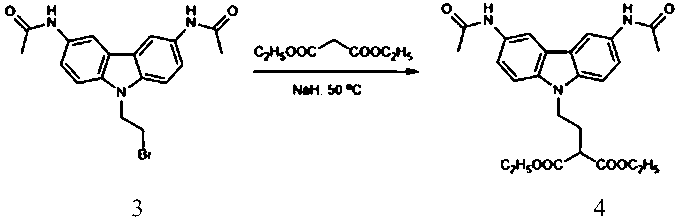
S4. 2.05g of compound 4 was dissolved in 50 mL of ethanol, 25 mL of a 3M aqueous solution of NaOH was added, and the mixture was heated through reflux; after reacting for 20 hours, the mixture was neutralized to pH=1 with 1M dilute hydrochloric acid, and a white precipitate was filtered and washed sequentially with water and ice ethanol to obtain pure compound 5 (1.77g, 98%), with ¹H NMR (400 MHz, DMSO-d6) *δ* 13.82 (s, 2H), 10.11 (s, 2H), 7.89-7.60 (m, 6H), 4.18-4.10 (m, 6H), 3.35-3.27 (m, 1H), 2.06 (m, 8H) measured; and a chemical equation of the hydrolysis reaction of compound 4 is as follows:

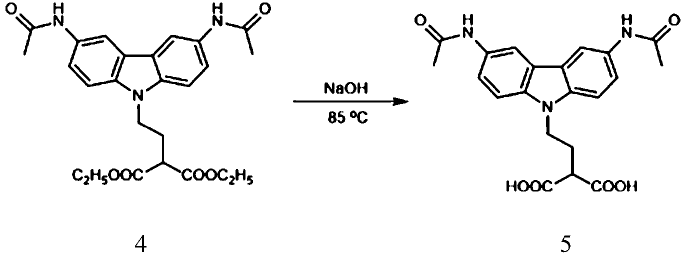

### Example A2:

This example provides an organic compound and a preparation method thereof. The organic compound in this example is denoted as the following chemical formula 10.

A preparation method of the organic compound denoted as chemical formula 10 includes the following steps:
S1. 1.6g of compound 6 and 14 mg of 1,1'-bis(diphenylphosphino)ferrocene were dissolved in 20 mL of N,N-dimethylformamide and 2 mL of water, and stirred uniformly, and then a solution of 84 mg of zinc acetate dihydrate, 13 mg of zinc powder, 10 mg of tris(dibenzylideneacetone)dipalladium (0), and at least 0.8g of zinc cyanide in N,N-dimethylformamide (20 mL) were added. The solution was stirred at 100°C for 72 hours, then added into 50 mL of an aqueous saturated sodium chloride solution and extracted three times with 50 mL of ethyl acetate; and a yellow oily liquid obtained after removal of the solvent was separated by silica gel column chromatography to obtain compound 7 (0.98g, 92%), with ¹H NMR (400 MHz, DMSO-d6) *δ* 11.66 (s, 1H), 7.81-7.75 (m, 4H), 7.33 (d, *J* = 7.8, 2H) measured; and a chemical equation of compound 6 and zinc cyanide is as follows: 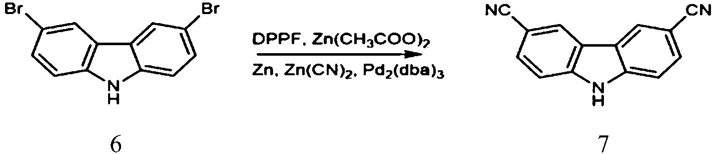
S2. Compared with step S2 in Example A1, 2.75g of compound 2 was replaced with 0.98g of compound 7, 1.85g of 1,2-dibromoethane was replaced with 1.33g of 2-bromomethyl-1,3-dibromopropane, and 1.52g of potassium carbonate was replaced with 0.77g of finally obtained compound 8 (1.53g, 78%), with ¹H NMR (400 MHz, CDCl3) *δ* 8.00-7.61 (m, 4H), 7.37 (d, *J* = 7.8, 2H), 4.03 (d, *J =* 7.2, 2H), 3.22 (d, *J =* 7.8, 4H), 2.35-2.26 (m, 1H) measured; and a chemical equation of compound 7 and 2-bromomethyl-1,3-dibromopropane is as follows: 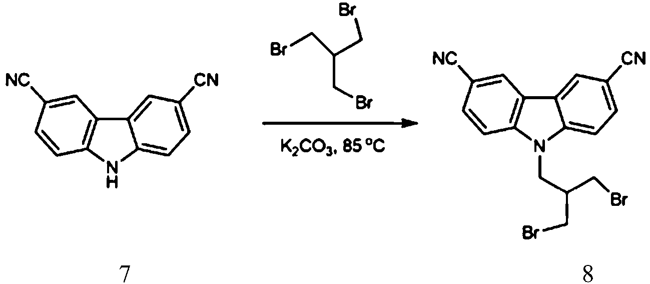
S3. Compared with step S3 in Example A1, the mass of NaH was changed from 0.36g to 0.38g, the mass of diethyl malonate was changed from 1.04g to 1.14g, and 2.54g of compound 3 was replaced with 1.53g of compound 8, to finally obtain compound 9 (2.03g, 97%), with ¹H NMR (400 MHz, CDCl3) *δ* 8.00-7.61 (m, 4H), 7.37 (d, *J =* 7.8, 2H), 4.16-4.12 (m, 8H), 4.03 (d, *J =* 7.2, 2H), 3.32-3.30 (m, 4H), 1.98-1.92 (m, 4H), 1.32-1.28 (m, 1H), 1.22-1.20 (m, 12H) measured; and a chemical equation of compound 8 and diethyl malonate is as follows: 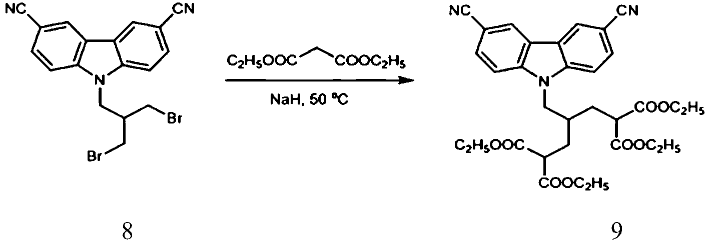
S4. Compared with step S4 in Example A1, 2.05g of compound 4 was replaced with 2.03g of compound 9, to finally obtain compound 10 (1.61g, 98%), with ¹H NMR (400 MHz, DMSO-d6) *δ* 13.82 (s, 1H), 8.00-7.61 (m, 4H), 7.37 (d, *J =* 7.8, 2H), 4.03 (d, *J =* 7.2, 2H), 3.42-3.36 (m, 2H), 1.73-1.69 (m, 4H), 1.32-1.28 (m, 1H) measured; and a chemical equation of the hydrolysis reaction of compound 9 is as follows:

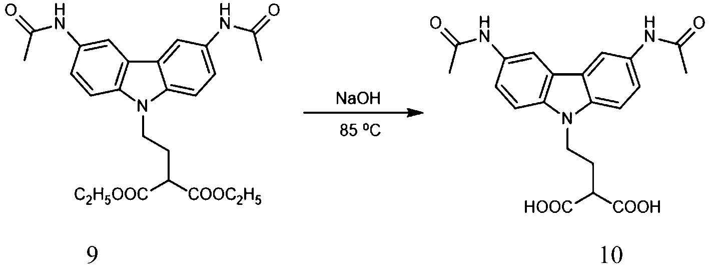

### Example A3:

This example provides an organic compound and a preparation method thereof. The organic compound in this example is denoted as the following chemical formula 14.

A preparation method of the organic compound denoted as chemical formula 14 includes the following steps:
S1. Compared with step S2 in Example A1, 2.75g of compound 2 was replaced with 2.07g of compound 11, 1.85g of 1,2-dibromoethane was replaced with 3.87g of 2,2-dibromomethyl-1,3-dibromopropane, and the mass of potassium carbonate was changed from 1.52g to 1.38g, to obtain compound 12 (3.86g, 75%) after reaction, with ¹H NMR (400 MHz, CDCl3) *δ* 6.75 (s, 2H), 3.71 (s, 2H), 3.16 (s, 6H), 2.37 (s, 6H) measured; and a chemical equation of compound 11 and 2,2-dibromomethyl-1,3-dibromopropane is as follows: 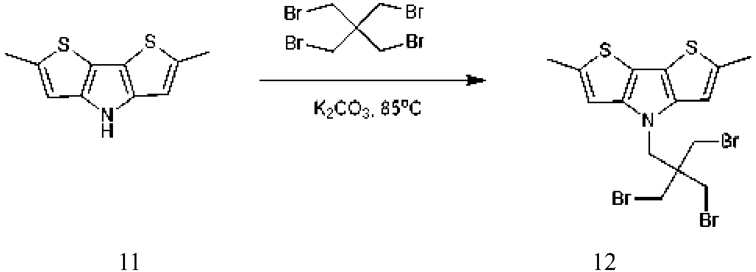
S2. Compared with step S3 in Example A1, the mass of NaH was changed from 0.36g to 1.17g, the mass of diethyl malonate was changed from 1.04g to 3.6g, and 2.54g of compound 3 was replaced with 3.86g of compound 12, to obtain compound 13 (3.76g, 66%) after reaction, with ¹H NMR (400 MHz, CDCl3) *δ* 6.75 (s, 2H), 4.18-4.10 (m, 12H), 3.71 (s, 2H), 3.34-3.28 (m, 3H), 3.16 (s, 6H), 2.37 (s, 6H), 1.91-1.87 (m, 6H), 1.23-1.19 (m, 9H) measured. A chemical equation of compound 12 and diethyl malonate is as follows: 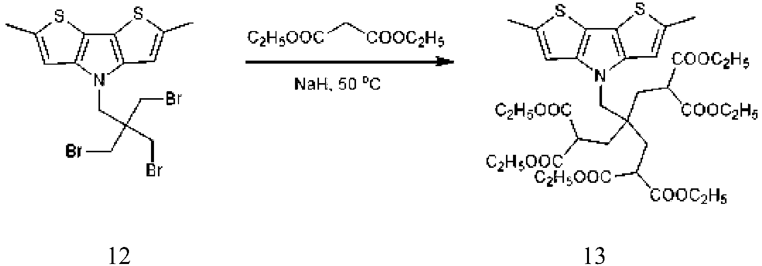
S3. Compared with step S4 in Example A1, 2.05g of compound 4 was replaced with 3.76g of compound 13, to obtain compound 14 (2.87g, 99%) after reaction, with ¹H NMR (400 MHz, DMSO-*d*6) *δ* 13.82 (s, 3H), 6.75 (s, 2H), 3.71 (s, 2H), 3.71 (s, 6H), 3.42-3.26 (m, 3H), 2.37 (s, 6H), 1.67-1.63 (m, 6H) measured; and a chemical equation of the hydrolysis reaction of compound 13 is as follows:

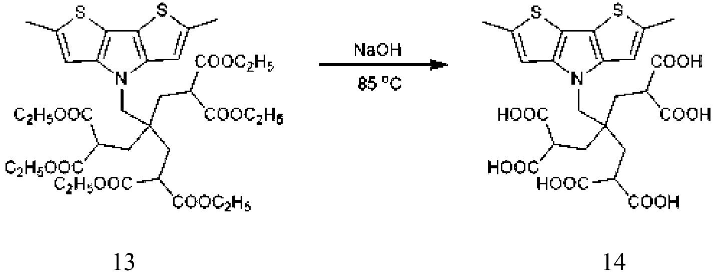

### Example A4:

This example provides an organic compound and a preparation method thereof. The organic compound in this example is denoted as the following chemical formula 18.

A preparation method of the organic compound denoted as chemical formula 18 includes the following steps:
S1. Compared with step S2 in Example A1, 2.75g of compound 2 was replaced with 2.06g of compound 15, the mass of 1,2-dibromoethane was changed from 1.85g to 3.7g, and the mass of potassium carbonate was changed from 1.52g to 3.04g, to obtain compound 16 (3.15g, 75%) after reaction, with ¹H NMR (400 MHz, CDCl3) *δ* 6.74 (s, 2H), 3.54-3.48 (m, 4H), 2.37 (s, 6H), 2.19-2.15 (m, 4H) measured; and a chemical equation of compound 15 and 1,2-dibromoethane is as follows: 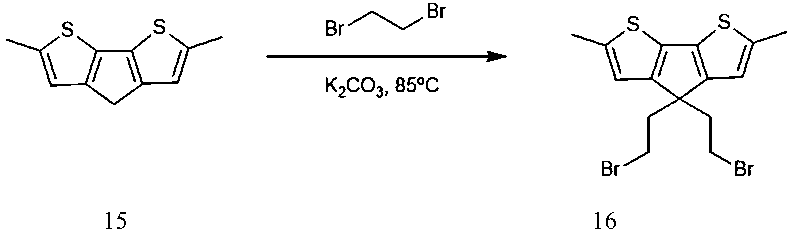
S2. Compared with step S3 in Example A1, the mass of NaH was changed from 0.36g to 0.72g, the mass of diethyl malonate was changed from 1.04g to 2.08g, and 2.54g of compound 3 was replaced with 3.15g of compound 16, to obtain compound 17 (2.75g, 66%) after reaction, with ¹H NMR (400 MHz, CDCl3) *δ* 6.74 (s, 2H), 4.18-4.10 (m, 8H), 3.34-3.28 (m, 2H), 2.37 (s, 6H), 1.80-1.64 (m, 8H), 1.24-1.18 (m, 12H) measured; and a chemical equation of compound 16 and diethyl malonate is as follows: 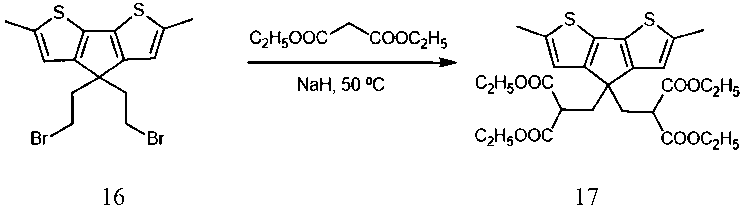
S3. Compared with step S4 in Example A1, 2.05g of compound 4 was replaced with 2.75g of compound 17, to obtain compound 18 (2.01g, 97%) after reaction, with 1H NMR (400 MHz, DMSO-*d*6) *δ* 13.87 (s, 4H), 6.74 (s, 2H), 3.42-3.36 (m, 2H), 2.37 (s, 6H), 1.68-1.52 (m, 8H) measured; a chemical equation of the hydrolysis reaction of compound 17 is as follows:

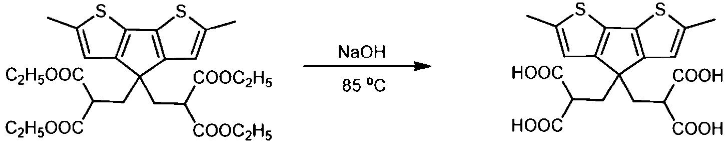

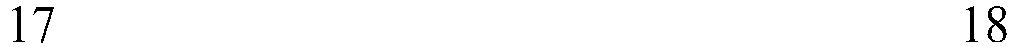

### Example A5:

This example provides an organic compound and a preparation method thereof. The organic compound in this example is denoted as the following chemical formula 21.

A preparation method of the organic compound denoted as chemical formula 21 includes the following steps:
S1. 2.29g of compound 19 and 3.01g of vinyl-1,1-di(diethyl phosphite) were dissolved in 50 mL of chloroform, and stirred under reflux for 6 hours to obtain compound 20 (5.21g, 97%), with ¹H NMR (400 MHz, CDCl3) *δ* 7.18 (d, *J* = 7.8, 4H), 6.79 (d, *J* = 7.8*,* 4H), 4.21-4.17 (m, 8H), 3.81 (s, 6H), 3.50 (d, *J* = 7.5, 2H), 1.42-1.34 (m, 13H) measured; and a chemical equation of compound 19 and vinyl-1,1-di(diethyl phosphite) is as follows: 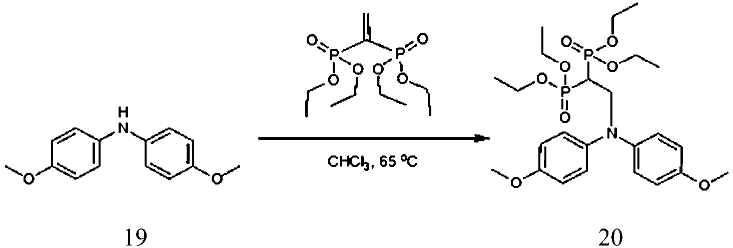
S2. 2.65g of the foregoing compound 20 was added to 50 mL of 1,4-dioxane, and 0.92g of trimethylsilyl bromide was added dropwise; after 22 hours of stirring at room temperature, 10 mL of methanol was added, the mixture was further stirred for 3 hours, and then distilled water (10 mL) was added dropwise until the solution became opaque, and the mixture was stirred overnight; the product was filtered out, washed with water, and dissolved in tetrahydrofuran until the product was completely dissolved, and n-hexane was added until no further precipitate formed; and the product was filtered out and washed with n-hexane to obtain compound 21 (2.09g, 73%), with ¹H NMR (400 MHz, DMSO-*d*6) *δ* 7.18 (d, *J =* 7.8, 4H), 6.79 (d, *J* = 7.8, 4H), 4.80 (s, 4H), 3.81 (s, 6H), 3.54 (d, *J* = 7.5, 2H), 1.42-1.38 (m, 1H) measured. A chemical equation of the hydrolysis reaction of compound 20 is as follows:

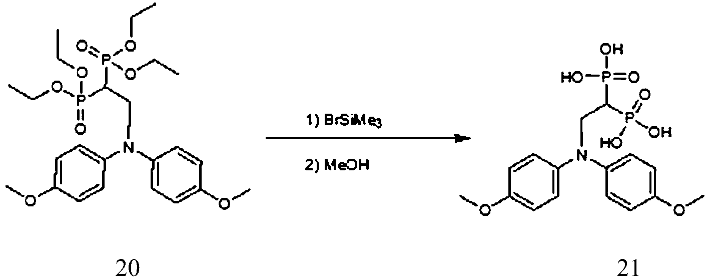

### Example A6:

This example provides an organic compound and a preparation method thereof. The organic compound in this example is denoted as the following chemical formula 24.

A preparation method of the organic compound denoted as chemical formula 24 includes the following steps:
S1. 3.83g of compound 22 was dissolved in 50 mL of tetrahydrofuran, 4 mL of 2.5M n-butyl lithium was slowly added dropwise at -78°C, the mixture was stirred for 2 hours, and then 3.01g of vinyl-1,1-di(diethyl phosphite) was slowly added dropwise; and after being warmed to room temperature, the mixture was stirred overnight to obtain compound 23 (5.01g, 79%), with ¹H NMR (400 MHz, CDCl3) *δ* 7.18 (d, *J* = 7.8, 6H), 7.03 (d, *J =* 7.8, 6H), 6.79 (d, *J* = 7.8, 4H), 4.20-4.16 (m, 8H), 3.81 (s, 6H), 2.87 (d, *J* = 7.5, 2H), 1.93-1.87 (m, 1H), 1.38-1.34 (m, 12H) measured; and a coupling reaction equation of compound 22 and vinyl-1,1-di(diethyl phosphite) is as follows: 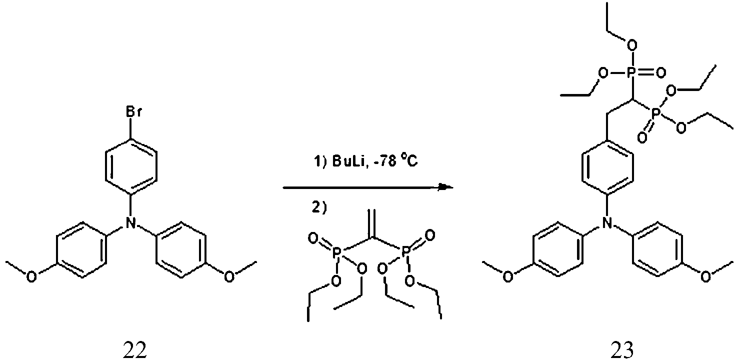
S2. 3.03g of the foregoing compound 23 was added to 50 mL of 1,4-dioxane, and 0.92g of trimethylsilyl bromide was added dropwise; after 22 hours of stirring at room temperature, 10 mL of methanol was added, the mixture was further stirred for 3 hours, and then distilled water (10 mL) was added dropwise until the solution became opaque, and the mixture was stirred overnight; the product was filtered out, washed with water, dissolved in tetrahydrofuran until the product was completely dissolved, and n-hexane was added until no further precipitate formed; and the product was filtered out and washed with n-hexane to obtain compound 24 (2.09g, 73%), with ¹H NMR (400 MHz, DMSO-*d*6) *δ* 7.18 (d, *J* = 7.8, 6H), 7.03 (d, *J* = 7.8, 6H), 6.79 (d, *J* = 7.8, 4H), 4.80 (s, 4H), 3.81 (s, 6H), 2.91 (d, *J* = 7.5, 2H), 1.93-1.87 (m, 1H) measured. A chemical equation of the hydrolysis reaction of compound 23 is as follows:

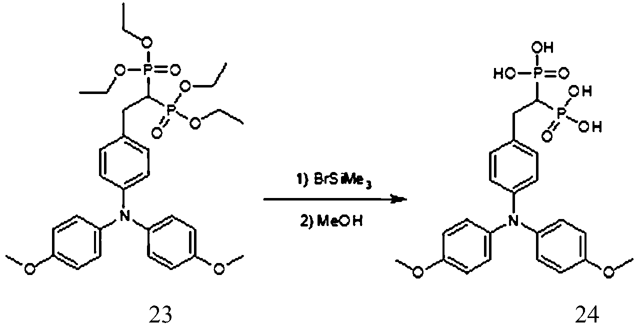

### Example A7:

This example provides an organic compound and a preparation method thereof. The organic compound in this example is denoted as the following chemical formula 25.

A preparation method of the organic compound denoted as chemical formula 25 includes the following steps:
S1. 1.04g of compound 21 was dissolved in 30 mL of anhydrous ethanol, 19.5 mL of a 1M aqueous solution of sodium hydroxide was added dropwise, and after the reaction completed, compound 25 (1.08g, 99%) was obtained, with ¹H NMR (400 MHz, DMSO-*d*6) *δ* 7.18 (d, *J =* 7.8, 4H), 6.79 (d, *J =* 7.8, 4H), 4.80 (s, 3H), 3.81 (s, 6H), 3.67-3.42 (m, 2H), 1.42-1.38 (m, 1H) measured; and a chemical equation for the preparation of compound 25 is as follows:

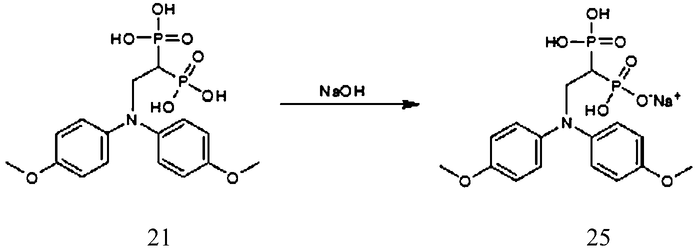

### Example A8:

This example provides an organic compound and a preparation method thereof. The organic compound in this example is denoted as the following chemical formula 26.

A preparation method of the organic compound denoted as chemical formula 26 includes the following steps:
S1. 1.04g of compound 21 was dissolved in 30 mL of anhydrous ethanol, 0.2g of piperidine was added dropwise, and after the reaction completed, compound 26 (1.22g, 98%) was obtained, with ¹H NMR (400 MHz, DMSO-*d*6) *δ* 9.79 (d, *J* = 7.8, 2H), 9.66 (d, *J* = 7.8, 2H), 7.18 (d, *J* = 7.8, 4H), 6.79 (d, *J* = 7.8, 4H), 4.80 (s, 3H), 3.81 (s, 6H), 3.67-3.42 (m, 2H), 1.42-1.38 (m, 1H) measured; and a chemical equation for the preparation of compound 26 is as follows:

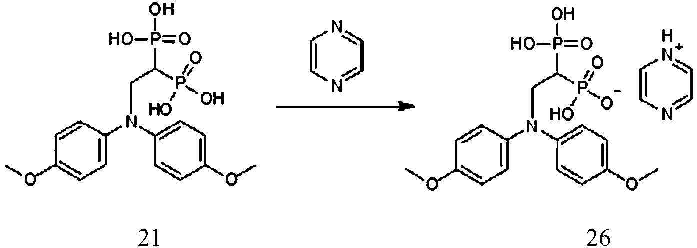

### Example A9:

This example provides an organic compound and a preparation method thereof. The organic compound in this example is denoted as the following chemical formula 30.

A preparation method of the organic compound denoted as chemical formula 30 includes the following steps:
S1. 3.71g of compound 27, 3.28g of compound 28, 0.59g of tetrakis(triphenylphosphine)palladium (Pd(PPh₃)₄), 50 mL of toluene, and 50 mL of a 2M aqueous solution of potassium carbonate (K₂CO₃) were added to a reaction flask, and the mixture was heated at 110°C for 48 hours and purified after reaction to obtain compound 29 (3.21g, 63%), with ¹H NMR (400 MHz, CDCl3) *δ* 7.62 (d, *J* = 7.8, 2H), 7.55 (d, *J* = 7.8, 2H), 7.37 (d, *J* = 7.8, 2H), 7.32 (d, *J* = 7.8, 2H), 7.24 (t, *J* = 7.4, 4H), 7.08 (d, *J* = 7.8, 4H), 7.04-6.99 (m, 2H), 4.10-4.06 (m, 4H), 3.83 (s, 1H), 3.42 (d, *J* = 7.5, 2H), 1.23-1.17 (m, 6H) measured; and a coupling reaction equation of compound 27 and compound 28 is as follows: 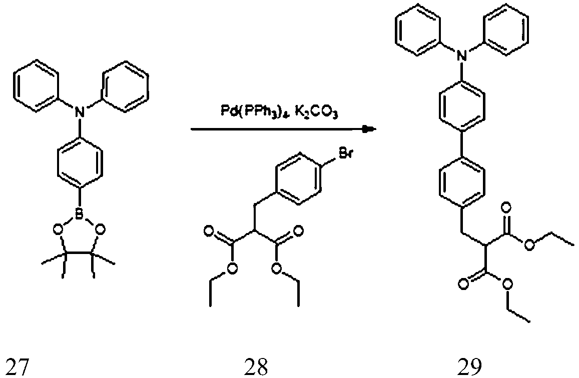
S2. 2.45g of compound 29, 20 mL of tetrahydrofuran, 20 mL of ethanol, and 20 mL of a 2M aqueous solution of sodium hydroxide (NaOH) were added to a reaction flask, the mixture was stirred at 75°C for 20 hours, then 20 mL of a 3M hydrochloric acid (aqueous solution of HCl) was added, and the product was filtered out to obtain compound 30 (2.09g, 73%), with ¹H NMR (400 MHz, DMSO-*d*6) *δ* 12.72 (s, 2H), 7.61 (d, *J =* 7.8, 2H), 7.54 (d, *J =* 7.8, 2H), 7.35 (d, *J* = 7.8, 2H), 7.32 (d, *J =* 7.8, 2H), 7.22 (t, *J* = 7.4, 4H), 7.07 (d, *J* = 7.8, 4H), 7.06-6.98 (m, 2H), 4.10-4.06 (m, 4H), 3.91-3.89 (m, 1H), 3.18 (d, *J* = 7.5, 2H) measured. A chemical equation of the hydrolysis reaction of compound 29 is as follows:

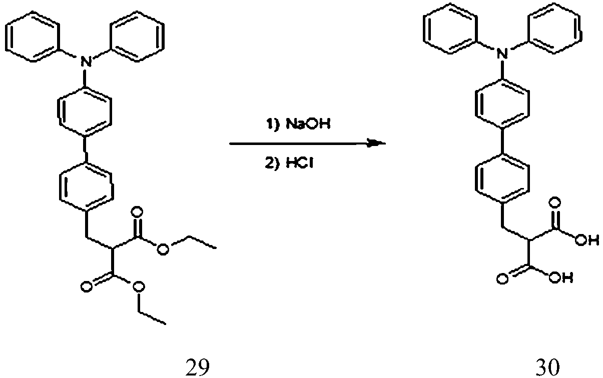

### Comparative Example A1

This comparative example provides an organic compound and a preparation method thereof. The organic compound in this example is denoted as the following chemical formula d4.

A preparation method of the organic compound denoted as chemical formula d4 includes the following steps:
S1 was the same as step S1 in Example A1.
S2 was the same as step S2 in Example A1.
S3. Compared with step S3 in Example A1, a solution of 0.49g of sodium cyanide in dimethyl sulfoxide was added to 0.27g of 18-crown-6 (18-crown 6-ether, C₁₂H₂₄O₆) and 0.14g of potassium carbonate, the mixture reacted with 2.54g of compound 3 at 100°C for 72 hours, then the reaction solution was added into an aqueous saturated sodium chloride solution and extracted three times with ethyl acetate; and a yellow oily liquid was obtained after removal of the solvent and separated by silica gel column chromatography to obtain compound d1 (0.74g, 37%), with ¹H NMR (400 MHz, DMSO-*d*6) *δ* 10.11 (s, 2H), 7.86 (d, *J* = 7.8, 4H), 7.59 (d, *J* = 7.8, 4H), 4.18-4.14 (m, 2H), 2.06 (s, 6H), 1.93-1.87 (m, 2H) measured; and a chemical equation of compound 3 and sodium cyanide is as follows: 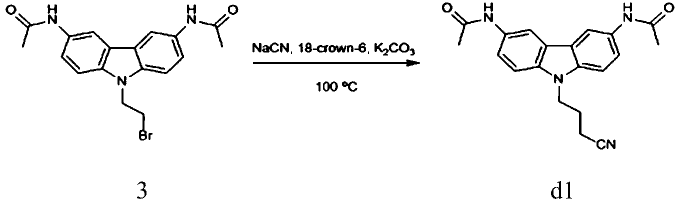
S3. Compared with step S4 in Example A1, compound 4 was replaced with compound d1 to obtain compound d2, with ¹H NMR (400 MHz, DMSO-d6) *δ* 12.01 (s, 1H), 10.11 (s, 2H), 7.86 (d, *J* = 7.8, 4H), 7.59 (d, *J* = 7.8, 4H), 4.14-4.10 (m, 2H), 2.52-2.48 (m, 2H), 2.06 (s, 6H) measured; and a chemical equation of the hydrolysis reaction of compound d1 is as follows:

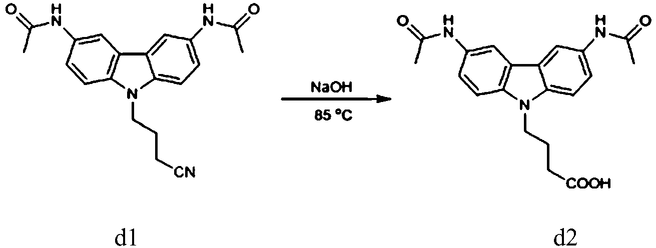

### 2. Examples of Perovskite Solar Cell and Preparation Method Thereof:

### Example B1:

This example provides a perovskite solar cell and a preparation method thereof. The perovskite solar cell in this example included a first electrode (transparent conductive oxide electrode), a hole transport layer, a perovskite layer, an electron transport layer, a blocking layer, and a second electrode sequentially stacked. The transparent conductive oxide electrode was FTO, a material of the hole transport layer was compound 4 in Example A1, a material of the perovskite layer was a CsFA system, a material of the electron transport layer was PC₆₁BM, a material of the blocking layer was BCP, and a material of the second electrode was Cu.

A preparation method of the perovskite solar cell includes the following steps:
S1. Pretreatment of the transparent conductive oxide electrode:
S11. 20 pieces of 2.0*2.0 cm FTO conductive glass were taken, and FTO at two end was etched by 0.35 cm via laser etching, to expose a glass substrate.
S12. The etched FTO conductive glass was ultrasonically cleaned sequentially with water, acetone, and isopropanol several times.
S13. The FTO conductive glass was dried with a nitrogen spray gun to remove the solvent and placed in an ultraviolet ozone cleaner for further cleaning.
S2. Compound 5 in Example A1 was dissolved in methanol to obtain a self-assembled molecule solution at a concentration of 0.3 mg/mL after dissolution; and the self-assembled molecules were spin-coated on a surface of the FTO substrate treated with ultraviolet ozone at 3000 rpm/s, and a 5 nm thick self-assembled molecular layer was obtained by vacuum drying or annealing.
S3. 726 mg of lead iodide, 240 mg of formamidinium iodide, 19 mg of cesium iodide, and 11 mg of lead bromide were taken and dissolved in 1 mL of a mixed solution of DMF and DMSO (a volume ratio of DMF to DMSO was 4:1), and the mixture was stirred for 3 hours and filtered with a 0.22 µm organic filter membrane to obtain a perovskite precursor solution; the perovskite precursor solution was spin-coated on a surface of the obtained self-assembled molecular layer at 3000 rpm, and the obtained self-assembled molecular layer was annealed at 100°C for 30 minutes and cooled to room temperature to form a 800 nm thick perovskite layer; and an active substance of the perovskite absorption layer was the CsFA system.
S4. A 20 nm thick electron transport layer PC₆₁BM was spin-coated on a surface of the perovskite layer at 1500 rpm/s, and the electron transport layer was annealed at 100°C for 10 minutes, and then immediately subjected to spin-coating at 5000 rpm to form a 5 nm thick passivation layer BCP.
S5. The obtained piece was placed in an evaporation deposition machine, and a 100 nm thick metal electrode Cu was deposited on a surface of the passivation layer by evaporation to obtain the perovskite solar cell, denoted as cell 1.

### Examples B2 to B8:

Examples B2 to B8 each provide a perovskite solar cell and a preparation method thereof. In Examples B2 to B8, compared with the perovskite solar cell in Example B1, a material of a hole transport layer contained in the perovskite solar cell in Example B2 was a material of chemical formula 10 in Example A2, a material of a hole transport layer contained in the perovskite solar cell in Example B3 was a material of chemical formula 14 in Example A3, a material of a hole transport layer contained in the perovskite solar cell in Example B4 was a material of chemical formula 18 in Example A4, and so on. By analogy, a material of a hole transport layer contained in the perovskite solar cell in Example B8 was a material of chemical formula 26 in Example A8.

In the preparation methods of the perovskite solar cells in Examples B2 to B8, compared with the preparation method of the perovskite solar cell in Example B1, in step S2 in the preparation method of the perovskite solar cell in Example B2, compound 10 in Example A2 was dissolved in methanol to form a hole transport layer, and other steps were unchanged; in step S2 in the preparation method of the perovskite solar cell in Example B3, compound 14 in Example A3 was dissolved in methanol to form a hole transport layer, and other steps were unchanged; and in step S2 in the preparation method of the perovskite solar cell in Example B4, compound 18 in Example A4 was dissolved in methanol to form a hole transport layer, and other steps were unchanged, and so on. By analogy, in step S2 in the preparation method of the perovskite solar cell in Example B8, compound 26 in Example A8 was dissolved in methanol to form a hole transport layer, and other steps were unchanged.

### Comparative Example B1:

Compared with the perovskite solar cell in Example B1, the perovskite solar cell in this Comparative Example B1 did not contain a hole transport layer, and other structural layers were unchanged.

Compared with the preparation method of the perovskite solar cell in Example B1, step S2 in the preparation method of the perovskite solar cell in Comparative Example B1 was not performed.

### Comparative Example B2

This comparative example provides a perovskite solar cell. Compared with Example B1, other materials were unreplaced except that a material of a hole transport layer contained in the perovskite solar cell in this comparative example was the organic compound d2 in the preparation method in Comparative Example A2.

Therefore, a difference between preparation methods of the perovskite solar cell in this comparative example and the perovskite solar cell in Example B1 was that compound 5 in step S2 of the preparation method of the perovskite solar cell in Example B1 was replaced with the foregoing organic compound d2, which was dissolved in methanol to form a hole transport layer, and other steps were unchanged.

### 3. Performance Test of Perovskite Solar Cell:

The perovskite solar cells in Examples B1 to B8 and Comparative Examples B1 to B2 were separately subjected to photoelectric conversion efficiency tests shown in Table 1 below.

In the photoelectric conversion efficiency test (I-V test) of the perovskite solar cells, a solar simulator (Enlitech) meeting Chinese National Standard IEC61215 was used; light intensity was calibrated by using a crystalline silicon solar cell to reach one solar intensity, AM 1.5; and the cells were connected to a digital source meter, and photoelectric conversion efficiency was measured under illumination.

Results of the I-V test are shown in Table 1 below:

**Table 1**

| Example | Material of hole transport layer | Photoelectric conversion efficiency | |
|---|---|---|---|
| | | Efficiency on Day 3 | Efficiency on Day 30 |
| Example B1 | Compound 5 in Example A1 | 24.01% | 23.52% |
| Example B2 | Compound 10 in Example A2 | 24.88% | 24.01% |
| Example B3 | Compound 14 in Example A3 | 24.56% | 24.01% |
| Example B4 | Compound 18 in Example A4 | 24.43% | 23.98% |
| Example B5 | Compound 21 in Example A5 | 25.37% | 25.01% |
| Example B6 | Compound 24 in Example A6 | 25.66% | 23.39% |
| Example B7 | Compound 25 in Example A7 | 25.71% | 25.46% |
| Example B8 | Compound 26 in Example A8 | 24.88% | 22.89% |
| Example B9 | Compound 30 in Example A9 | 25.81% | 24.29% |
| Comparative Example B1 | None | 15.88% | 15.15% |
| Comparative Example B2 | Compound d2 in Comparative Example A1 | 23.29% | 22.25% |

It can be learned from the photoelectric conversion efficiency of each perovskite solar cell and stability data of the photoelectric conversion efficiency in Table 1 that, the photoelectric conversion efficiency of the perovskite solar cells in Examples B1 to B8 in this application is significantly higher than photoelectric conversion efficiency of the perovskite solar cell in Comparative Example B1, and also higher than photoelectric conversion efficiency of the perovskite solar cell in Comparative Example B2; and the perovskite solar cells in Examples B1 to B8 in this application have close photoelectric conversion efficiency on Day 30 and Day 3. Therefore, the perovskite solar cells in the examples of this application have significantly improved photoelectric conversion efficiency compared to self-assembled molecules with a single head group, and have stable photoelectric conversion performance.

It can be learned from the photoelectric conversion stability and photoelectric conversion efficiency of the perovskite solar cells in Examples B1 to B8 of this application that, the organic compound provided in the examples of this application can significantly enhance bonding strength, a bonding volume, and uniformity on the surface of the transparent conductive oxide electrode, which can significantly improve completeness of the monomolecular film layer formed by the tail group contained in the organic compound, to significantly improve a function exerted by the organic compound as the hole transport layer, thereby effectively improving match between the work function of the first electrode such as the transparent conductive oxide electrode and the energy level of the perovskite layer interface, reducing energy loss, increasing the open-circuit voltage of the perovskite cell, and significantly enhancing the photoelectric conversion efficiency of the perovskite solar cell.

Finally, it should be noted that the foregoing embodiments are merely used to illustrate the technical solutions of this application instead of limiting this application. Although this application is described in detail with reference to the foregoing embodiments, a person of ordinary skill in the art should understand that modifications can still be made to the technical solutions described in the foregoing embodiments, or equivalent replacements can be made to some or all of the technical features thereof, without departing from the scope of the technical solutions of the embodiments of this application, which all fall within the scope of the claims and specification of this application. In particular, as long as no structural conflict exists, the technical features mentioned in each embodiment can be combined in any manner. This application is not limited to the specific embodiments disclosed in this specification but includes all technical solutions falling within the scope of the claims.

## Claims

1. An organic compound, comprising head groups, a tail group, and a carbon chain, wherein the carbon chain connects the head groups to the tail group, and there are at least two head groups.

2. The organic compound according to claim 1, **characterized in that** the at least two head groups are identical or different and comprise at least two of -SO₃H, -PO(OH)₂, -COOH, and -Si(ORₐ), or at least two salts of -SO₃H, -PO(OH)₂, and -COOH, wherein Rₐ is a first alkyl group.

3. The organic compound according to claim 1 or 2, **characterized in that** the tail group comprises at least one group selected from substituted or unsubstituted carbazole, substituted or unsubstituted cyclopentadithiophene, substituted or unsubstituted benzodithiophene, substituted or unsubstituted pyrrolodithiophene, substituted or unsubstituted diphenylamine, substituted or unsubstituted triphenylamine, and substituted or unsubstituted triphenylmethane.

4. The organic compound according to claim 3, **characterized in that** the carbazole group is 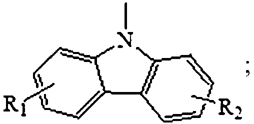
the cyclopentadithiophene group is 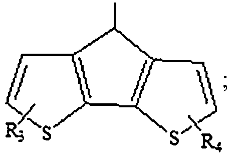 the benzodithiophene group is 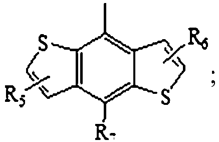 the pyrrolodithiophene group is 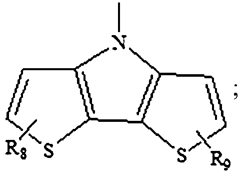 the diphenylamine group is 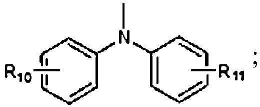 and
the triphenylamine group is 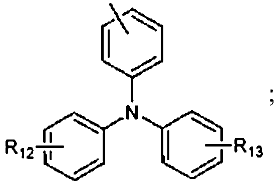
wherein R₁, R₂, R₃, R₄, R₅, R₆, R₇, R₈, R₉, R₁₀, R₁₁, R₁₂, and R₁₃ independently comprise any one of hydrogen, halogen, -O-R_{b}, -OH, -NHCOR_{c}, -OCOR_{d}, -CH₂COOH, -Rₑ, a phenyl group, a halogen-substituted phenyl group, halogen-substituted R_{f}, a nitrogen-containing group, nitrogen-containing group-substituted R_{g}, and a nitrogen-containing group-substituted phenyl group, wherein R_{b}, R_{c}, R_{d}, Rₑ, R_{f}, and R_{g} independently represent a second alkyl group; and/or
the first alkyl group is a C1-C5 alkyl group.

5. The organic compound according to claim 4, **characterized in that** the second alkyl group is a C1-C5 alkyl group; and/or
the nitrogen-containing group comprises any one of -N(Rₕ)₂, -NHRᵢ, -NH₂, a trimethylamino group, a triethylamino group, and a tripropylamino group, wherein Rₕ and Rᵢ independently represent a third alkyl group.

6. The organic compound according to any one of claims 1 to 5, **characterized in that** the carbon chain comprises at least one of an alkyl chain and a heteroatom-containing alkyl chain; and/or
one end of the carbon chain is connected to the head group, and another end of the carbon chain is connected to the tail group.

7. The organic compound according to claim 6, **characterized in that** the number of carbon atoms in the alkyl chain ranges from C1 to C10; and/or
the heteroatom comprises at least one of O, S, N, B, and Si.

8. The organic compound according to any one of claims 4 to 6, **characterized in that** the organic compound comprises at least one of the following compounds or salts of the compounds: 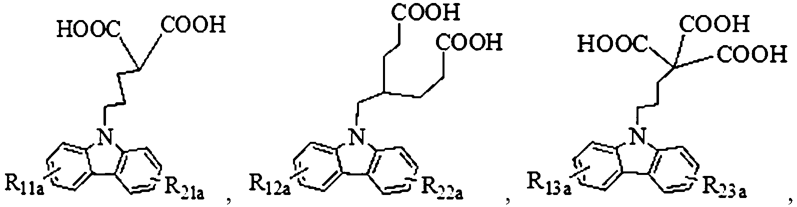 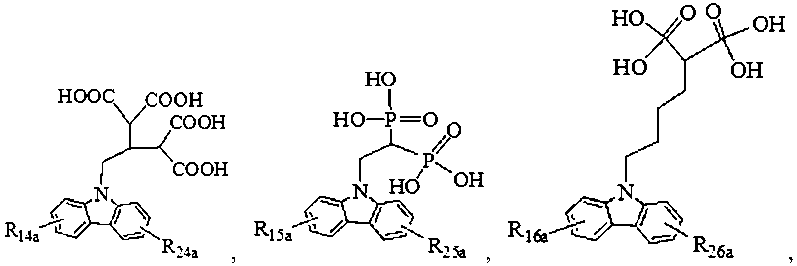 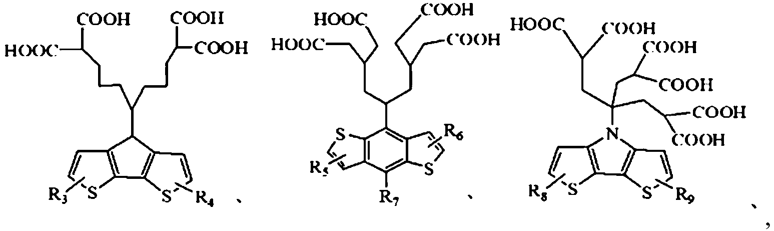 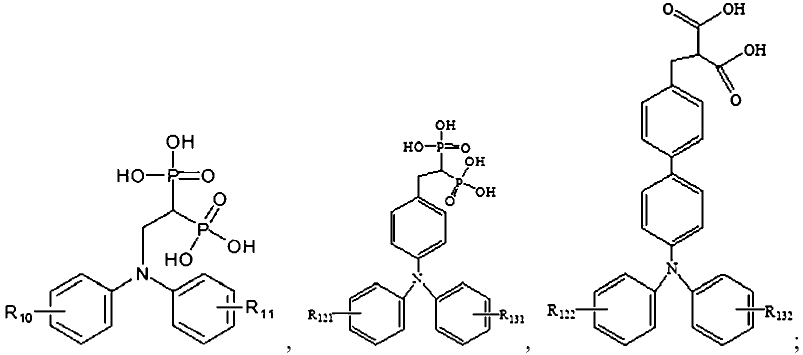 wherein R₁₁ₐ, R₁₂ₐ, R₁₃ₐ, R₁₄ₐ, R₁₅ₐ, and R₁₆ₐ independently represent R₁, R₂₁ₐ, R₂₂ₐ, R₂₃ₐ, R₂₄ₐ, R₂₅ₐ, and R₂₆ₐ independently represent R₂, R₁₂₁ and R₁₂₂ independently represent R₁₂, and R₁₃₁ and R₁₃₂ independently represent R₁₃.

9. A preparation method of the organic compound according to any one of claims 1 to 8, **characterized by** comprising the following steps:
subjecting a reactant Fₐ containing a tail group and a reactant F_{b} containing a first carbon chain to a coupling reaction, to connect the tail group to the first carbon chain to generate an intermediate product Cₐ; and
subjecting the intermediate product Cₐ and a reactant F_{c} containing a first ester group to a substitution reaction, wherein the first ester group is an ester group containing a first head group, so that the first head group is connected to the first carbon chain to generate an intermediate product C_{b};
or
subjecting a reactant Fₐ containing a tail group and a reactant Fₑ containing a second carbon chain and a second ester group to an addition reaction, to connect the tail group to the second carbon chain to generate an intermediate product C_{c}, wherein the second ester group is an ester group containing a second head group, and the second head group is connected to the second carbon chain; and
subjecting the first ester group contained in the intermediate product C_{b} and/or the second ester group contained in the intermediate product C_{c} to a hydrolysis reaction to generate a final product of the organic compound;
wherein the final product of the organic compound contains at least two head groups.

10. The preparation method according to claim 9, **characterized in that** the reactant Fₐ comprises at least one of the following compounds: 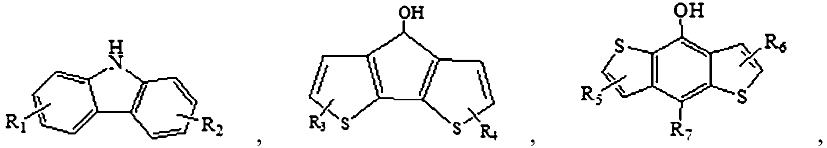 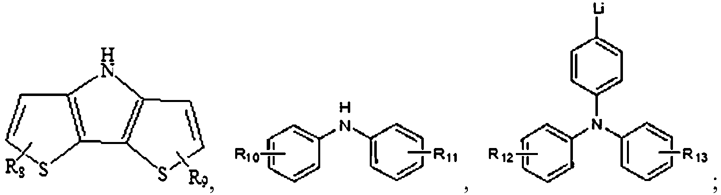
wherein R₁, R₂, R₃, R₄, R₅, R₆, R₇, R₈, R₉, R₁₀, R₁₁, R₁₂, and R₁₃ independently comprise any one of hydrogen, halogen, -O-R_{b}, -OH, -NHCOR_{c}, -OCORₐ, -CH₂COOH, -Rₑ, a phenyl group, a halogen-substituted phenyl group, halogen-substituted R_{f}, a nitrogen-containing group, nitrogen-containing group-substituted R_{g}, and a nitrogen-containing group-substituted phenyl group, wherein R_{b}, R_{c}, R_{d}, Rₑ, R_{f}, and R_{g} independently represent a fourth alkyl group; and/or
the reactant F_{b} comprises any one of a haloalkane compound, a heteroatom-containing haloalkane compound, and a halogenated alkyl acyl halide compound; and/or
the reactant F_{c} comprises a compound containing at least two of an ester group of -SO₃H, an ester group of -PO(OH)₂, an ester group of -COOH, and -Si(ORₐ); wherein Rₐ represents a fifth alkyl group; and/or
the reactant F_{c} comprises any one of a heteroatom-containing haloalkane compound and a halogenated compound containing at least one of an ester group of - SO₃H, an ester group of -PO(OH)₂, an ester group of -COOH, and -Si(ORₐ)₃ and any one of an alkyl chain and a heteroatom-containing alkyl chain, wherein Rₐ represents a sixth alkyl group.

11. The preparation method according to claim 9 or 10, **characterized in that** solvents for the coupling reaction and the substitution reaction independently comprise at least one of N,N-dimethylformamide, toluene, water, 1,2-xylene, chlorobenzene, 1,2-dichlorobenzene, tetrahydrofuran, and ethanol;
a solvent for the hydrolysis reaction comprises at least one of 1,4-dioxane, water, anhydrous ethanol, tetrahydrofuran, toluene, 1,2-xylene, chlorobenzene, and 1,2-dichlorobenzene; and/or
a solvent for the addition reaction comprises at least one of tetrahydrofuran, anhydrous ethanol, toluene, 1,2-xylene, chlorobenzene, and 1,2-dichlorobenzene; and/or
the reactant Fₑ comprises at least one of the following compounds: 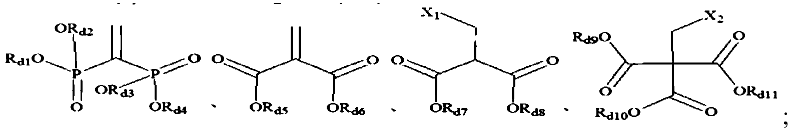
wherein R_{d1}, R_{d2}, R_{d3}, R_{d4}, R_{d5}, R_{d6}, R_{d7}, R_{d8}, R_{d9}, R_{d10}, and R_{d11} independently represent a C1-C5 alkyl chain, and X₁ and X₂ independently represent a halogen atom.

12. A hole transport material, **characterized in that** the hole transport material comprises the organic compound according to any one of claims 1 to 8 or the organic compound prepared in the preparation method according to any one of claims 9 to 11.

13. A perovskite solar cell, comprising a hole transport layer, **characterized in that** the hole transport layer comprises the organic compound according to any one of claims 1 to 8, the organic compound prepared in the preparation method according to any one of claims 9 to 11, or the hole transport material according to claim 12.

14. The perovskite solar cell according to claim 13, **characterized in that** a thickness of the hole transport layer ranges from 0.1 nm to 10 nm; and/or
the hole transport layer is stacked between a conductive oxide electrode and a perovskite layer contained in the perovskite solar cell.

15. The perovskite solar cell according to claim 13 or 14, **characterized in that** perovskite contained in a perovskite layer of the perovskite solar cell comprises at least one of ABX₃ and A₂CDX₆, wherein A, B, C, and D independently and differently represent an inorganic or organic cation or a mixture of organic and inorganic cations, and X represents an inorganic or organic anion or a mixture of organic and inorganic anions; and/or
a material of a conductive oxide electrode contained in the perovskite solar cell comprises at least one of a transparent material of a fluorine-doped tin oxide, indium tin oxide, a transparent conductive material of an aluminum-doped zinc oxide, a transparent conductive material of a boron-doped zinc oxide, and a transparent conductive material of an indium-doped zinc oxide.

16. A preparation method of a perovskite solar cell, **characterized by** comprising the following steps:
providing a first electrode;
preparing a slurry comprising an organic compound or a hole transport material, and subjecting the slurry to film-forming processing on a surface of the first electrode to form a hole transport layer;
forming a perovskite layer on a surface, facing away from the first electrode, of the hole transport layer;
forming an electron transport layer on a surface, facing away from the hole transport layer, of the perovskite layer; and
forming a second electrode on a surface, facing away from the perovskite layer, of the electron transport layer;
wherein the organic compound comprises the organic compound according to any one of claims 1 to 8 or the organic compound prepared in the preparation method according to any one of claims 9 to 11, and the hole transport material comprises the hole transport material according to claim 12.

17. The preparation method according to claim 16, **characterized in that** the first electrode is a conductive oxide electrode; and/or
a concentration of the organic compound or the hole transport material in the slurry ranges from 0.1 mg/mL to 10 mg/mL.

18. An electric apparatus, **characterized in that** the electric apparatus comprises the perovskite solar cell according to any one of claims 13 to 15 or the perovskite solar cell prepared in the preparation method according to any one of claims 16 and 17, wherein the perovskite solar cell serves as a power supply or an energy storage unit of the electric apparatus.
